# EUROPEAN PATENT APPLICATION

(11) **EP 1 365 030 A1**
(43) Date of publication of application: **26.11.2003**
(21) Application number: 02010273.7
(22) Date of filing: 21.05.2002
(51) Int. Cl.: C12Q 1/68, C07K 14/72, G01N 33/574, A61P 43/00, C07K 16/28

(54) **G-protein coupled receptor marker molecules associated with colorectal lesions**

(71) Applicant: MTM Laboratories AG, 69120 Heidelberg (DE)
(72) Inventor: Hipfel, Rainer, 69126 Heidelberg (DE); Coy, Johannes, 63762 Grossostheim (DE)

(57) **Abstract**

The present invention relates to nucleic acids and polypeptides associated with colorectal lesions. The invention is more specifically related to a nucleic acid encoding a G-protein coupled receptor (GPCR) and splicing variants thereof and the polypeptide transcribed thereof, the expression of which is significantly altered in association with colorectal cancer and other cancers. Another aspect of the nucleic acids disclosed herein are transcripts of the gene disclosed herein, that are differentially produced in tumors of the gastrointestinal tract. Futhermore the present invention provides a method for early diagnosis, prognosis and monitoring of the disease course and for therapy and vaccination of colorectal lesions.

SEQUENCE LISTING

<110>  MTM Laboratories AG

<120>  Marker Molecules associated with colorectal lesions

<130>  colma1/P/EP

<140>  02010273.7
<141>  2002-05-21

<160>  7

<170>  PatentIn version 3.1

<210>  1
<211>  2714
<212>  DNA
<213>  Homo sapiens

<220>
<221>  misc_feature
<222>  (606)..(607)
<223>  exon boundary


<220>
<221>  misc_feature
<222>  (808)..(809)
<223>  exon boundary


<220>
<221>  misc_feature
<222>  (885)..(886)
<223>  exon boundary


<220>
<221>  misc_feature
<222>  (972)..(973)
<223>  exon boundary


<220>
<221>  misc_feature
<222>  (1153)..(1154)
<223>  exon boundary


<220>
<221>  misc_feature
<222>  (1783)..(1788)
<223>  n: sequence containing three alternative splicing sites (length a
       pproximately 1.6kb, 2.6kb, 3.6kb, respectively)

<220>
<221> primer_bind
<222> (1024)..(1043)
<223> forward primer


<220>
<221> primer_bind
<222> (2509)..(2530)
<223> reverse primer


<400> 1
gtgatggata tctgcagaat tcgcccttaa gcagtggtaa acaacgcaga gtacgcggga 60

ggaggcaagc tggactccct cactcagctg caggagcaag gacagtgagg ctcaaccccg 120

cctgagccat gccagccaac ttcacagagg gcagcttcga ttccagtggg accgggcaga 180

cgctggattc ttccccagtg gcttgcactg aaacagtgac ttttactgaa gtggtggaag 240

gaaaggaatg gggttccttc tactactcct ttaagactga gcaattgata actctgtggg 300

tcctctttgt ttttaccatt gttggaaact ccgttgtgct tttttccaca tggaggagaa 360

agaagaagtc aagaatgacc ttctttgtga ctcagctggc catcacagat tctttcacag 420

gactggtcaa catcttgaca gatattattt ggcgattcac tggagacttc acggcacctg 480

acctggtttg ccgagtggtc cgctatttgc aggttgtgct gctctacgcc tctacctacg 540

tcctggtgtc cctcagcata gacagatacc atgccatcgt ctaccccatg aagttccttc 600

aaggagaaaa gcaagccagg gtcctcattg tgatcgcctg gagcctgtct tttctgttct 660

ccattcccac cctgatcata tttgggaaga ggacactgtc caacggtgaa gtgcagtgct 720

gggccctgtg gcctggcgac tcctactgga ccccatacat gaccatcgtg gccttcctgg 780

tgtacttcat ccctctgaca atcatcagca tcatgtatgg cattgtgatc cgaactattt 840

ggattaaaag caaaacctac gaaacagtga tttccaactg ctcagatggg aaactgtgca 900

gcagctataa ccgaggactc atctcaaagg caaaaatcaa ggctatcaag tatagcatta 960

tcatcattct tgccttcatc tgctgttgga gtccatactt cctgtttgac attttggaca 1020

atttcaacct ccttccagac acccaggagc gtttctatgc ctctgtgatc attcagaacc 1080

tgccagcatt gaatagtgcc atcaaccccc ccatctactg tgtcttcagc agctccatct 1140

ctttcccctg cagggagcaa agatcacagg attccagaat gacgttccgg gagagaactg 1200

agaggcatga gatgcagatt ctgtccaagc cagaattcat ctagacccta gggcagtgcc 1260

agtgctaggc tgagcaccat cagctctccc aggtccttgt cacctgcttg ggcacgtgca 1320

tggaacccga gccaacttca ccccaccctc gtcattacct gggagatgca caagacaaat 1380
gttctaatga ctgcatgcac tgcttaagta ttggccaaca cgaactcccc agttattcat 1440
gccagccagg aaggaaacgc cttccttccc caccattccc agccctcctt cccactggcc 1500
agcacctgaa cccagtgaac acaggcatta gtggtccagg gtcctggctt ggagccagtg 1560
agtagacagg caagcagagg ggacaaaggt agctgggtta tacatgaata ttctcattac 1620
aatagaagaa aataaaagac ttaattaagc ccatattttt cccccagttt tggattggag 1680
gttcagtgct tgtagccaag aaagtgtttg tccttgaaat gccaacaaat tcatttccag 1740
gcattggtgc tttgcacctc ttctgcagat agcctcggtt tgnnnnnnct aaaatagtag 1800
aaatgcaaaa acatacatct ttcagagtaa aagagaagga aagctgagtg ataagtcact 1860
caaacaattt gaaagaaagc aagaaaagag agaaacaggg acataaaaca gctgggataa 1920
acagaaaaca cataggaaga tggtggtttt tttatccaaa cttactagta attataagtg 1980
caaagagatt aaatgcccca gttaataatc aaagactatc agactgattt tttaaaaaat 2040
aaataactgt ctgctgttta caaaagacat gcctaaaaca caaggataca gaaagtttga 2100
aaatcaaaga atggatcaag gtagtccatg gaaactgtag ctaaaagaaa gctggtaagg 2160
ctatattaat aaagactaaa taccttttaa ggcaaaaagt attagtagag ttaaacaggg 2220
acacttcgta atgataaaga ttttagttta ataagatgaa gtaataattc taaatttcca 2280
ggcatttaaa aacatggcct caaaatatat acttttaaaa gttgtcagaa ctatgaaaca 2340
aatagaaaaa ttcacaaccc taatggggaa ttttatcaca cttaatgcca gtcattaaag 2400
cagacaaaaa tcaggaatga tctggataat ttgagcaaca tgaataacac aggaagagca 2460
ctttacctta ccacccctga acagacattt ttttcaagca cacatgaagc agttaccaaa 2520
gttgaccaca tgctaggcca taaaacaagc ttcaacactt ccacgagatt gaattcatat 2580
agtctagttt ttctgatcat gttataatta tgctggaact caagagtaag aggataacta 2640
gaaaattcaa ataggtttgg aagttaggaa atacacttcc gaacaactta ttgccaaaga 2700
agaaagcata tgaa 2714

<210> 2
<211> 371
<212> PRT
<213> Homo sapiens

<400> 2

Met Pro Ala Asn Phe Thr Glu Gly Ser Phe Asp Ser Ser Gly Thr Gly

1                   5                                                   15

Gln Thr Leu Asp Ser Ser Pro Val Ala Cys Thr Glu Thr Val Thr Phe
                20                  25                  30

Thr Glu Val Val Glu Gly Lys Glu Trp Gly Ser Phe Tyr Tyr Ser Phe
        35                  40                  45

Lys Thr Glu Gln Leu Ile Thr Leu Trp Val Leu Phe Val Phe Thr Ile
    50                  55                  60

Val Gly Asn Ser Val Val Leu Phe Ser Thr Trp Arg Arg Lys Lys Lys
65                  70                  75                  80

Ser Arg Met Thr Phe Phe Val Thr Gln Leu Ala Ile Thr Asp Ser Phe
                85                  90                  95

Thr Gly Leu Val Asn Ile Leu Thr Asp Ile Ile Trp Arg Phe Thr Gly
            100                 105                 110

Asp Phe Thr Ala Pro Asp Leu Val Cys Arg Val Val Arg Tyr Leu Gln
        115                 120                 125

Val Val Leu Leu Tyr Ala Ser Thr Tyr Val Leu Val Ser Leu Ser Ile
    130                 135                 140

Asp Arg Tyr His Ala Ile Val Tyr Pro Met Lys Phe Leu Gln Gly Glu
145                 150                 155                 160

Lys Gln Ala Arg Val Leu Ile Val Ile Ala Trp Ser Leu Ser Phe Leu
                165                 170                 175

Phe Ser Ile Pro Thr Leu Ile Ile Phe Gly Lys Arg Thr Leu Ser Asn
            180                 185                 190

Gly Glu Val Gln Cys Trp Ala Leu Trp Pro Gly Asp Ser Tyr Trp Thr
        195                 200                 205

Pro Tyr Met Thr Ile Val Ala Phe Leu Val Tyr Phe Ile Pro Leu Thr
    210                 215                 220

Ile Ile Ser Ile Met Tyr Gly Ile Val Ile Arg Thr Ile Trp Ile Lys
225                 230                 235                 240

Ser Lys Thr Tyr Glu Thr Val Ile Ser Asn Cys Ser Asp Gly Lys Leu
                245                 250                 255

Cys Ser Ser Tyr Asn Arg Gly Leu Ile Ser Lys Ala Lys Ile Lys Ala
            260                 265                 270

Ile Lys Tyr Ser Ile Ile Ile Ile Leu Ala Phe Ile Cys Cys Trp Ser
        275                 280                 285

Pro Tyr Phe Leu Phe Asp Ile Leu Asp Asn Phe Asn Leu Leu Pro Asp
    290                 295                 300

Thr Gln Glu Arg Phe Tyr Ala Ser Val Ile Ile Gln Asn Leu Pro Ala
305                 310                 315                 320

Leu Asn Ser Ala Ile Asn Pro Pro Ile Tyr Cys Val Phe Ser Ser Ser
                325                 330                 335

Ile Ser Phe Pro Cys Arg Glu Gln Arg Ser Gln Asp Ser Arg Met Thr
            340                 345                 350

Phe Arg Glu Arg Thr Glu Arg His Glu Met Gln Ile Leu Ser Lys Pro
        355                 360                 365

Glu Phe Ile
    370

<210>  3
<211>  2439
<212>  DNA
<213>  Homo sapiens

<220>
<221>  misc_feature
<222>  (606)..(607)
<223>  exon boundary

<220>
<221>  misc_feature
<222>  (808)..(809)
<223>  exon boundary

<220>
<221>  misc_feature

<222>  (885)..(886)
<223>  exon boundary


<220>
<221>  misc_feature
<222>  (972)..(973)
<223>  exon boundary


<220>
<221>  misc_feature
<222>  (1088)..(1089)
<223>  exon boundary


<220>
<221>  primer_bind
<222>  (1024)..(1043)
<223>  forward primer


<220>
<221>  primer_bind
<222>  (2234)..(2255)
<223>  reverse primer


<400>  3
gtgatggata tctgcagaat tcgcccttaa gcagtggtaa acaacgcaga gtacgcggga      60

ggaggcaagc tggactccct cactcagctg caggagcaag gacagtgagg ctcaaccccg     120

cctgagccat gccagccaac ttcacagagg gcagcttcga ttccagtggg accgggcaga     180

cgctggattc ttccccagtg gcttgcactg aaacagtgac ttttactgaa gtggtggaag     240

gaaaggaatg gggttccttc tactactcct ttaagactga gcaattgata actctgtggg     300

tcctctttgt ttttaccatt gttggaaact ccgttgtgct tttttccaca tggaggagaa     360

agaagaagtc aagaatgacc ttctttgtga ctcagctggc catcacagat tctttcacag     420

gactggtcaa catcttgaca gatattattt ggcgattcac tggagacttc acggcacctg     480

acctggtttg ccgagtggtc cgctatttgc aggttgtgct gctctacgcc tctacctacg     540

tcctggtgtc cctcagcata gacagatacc atgccatcgt ctaccccatg aagttccttc     600

aaggagaaaa gcaagccagg gtcctcattg tgatcgcctg gagcctgtct tttctgttct      660

ccattcccac cctgatcata tttgggaaga ggacactgtc caacggtgaa gtgcagtgct     720

gggccctgtg gcctggcgac tcctactgga ccccatacat gaccatcgtg gccttcctgg     780

tgtacttcat ccctctgaca atcatcagca tcatgtatgg cattgtgatc cgaactattt     840

ggattaaaag caaaacctac gaaacagtga tttccaactg ctcagatggg aaactgtgca 900

gcagctataa ccgaggactc atctcaaagg caaaaatcaa ggctatcaag tatagcatta 960

tcatcattct tgccttcatc tgctgttgga gtccatactt cctgtttgac attttggaca 1020

atttcaacct ccttccagac acccaggagc gtttctatgc ctctgtgatc attcagaacc 1080

tgccagcact ttgggaggcc gaggagggcg gatcacctga gttcgaacag gagttcgaga 1140

ccatcccagt caatatggtg aaaacccgtc tgtactaaaa atacaaaaat tagccgggta 1200

tggtggcggg ccgcctgtaa tctcagctac ttgggaggct gaggcaggag aatcacttaa 1260

acctgggagg aggaggttgc agtgagccga gatcgcacca ttgcactctg gcctgggcaa 1320

caagaaggaa actctgcctg gaaaaaaaaa aaacaaaaaa caaaaaaaac attattacgc 1380

acaacaatag catagaaatc gagaggcaat taaatgtagt tagtttcaag ttccttgaat 1440

tatcttgcat gcaaaatgtg gattattagt cattgataca ccaagaatgc atgttacaat 1500

ttcagggtca tcactaaaat agtagaaatg caaaaacata catctttcag agtaaaagag 1560

aaggaaagct gagtgataag tcactcaaac aatttgaaag aaagcaagaa aagagagaaa 1620

cagggacata aaacagctgg gataaacaga aaacacatag gaagatggtg gtttttttat 1680

ccaaacttac tagtaattat aagtgcaaag agattaaatg ccccagttaa taatcaaaga 1740

ctatcagact gattttttaa aaaataaata actgtctgct gtttacaaaa gacatgccta 1800

aaacacaagg atacagaaag tttgaaaatc aaagaatgga tcaaggtagt ccatggaaac 1860

tgtagctaaa agaaagctgg taaggctata ttaataaaga ctaaatacct tttaaggcaa 1920

aaagtattag tagagttaaa cagggacact tcgtaatgat aaagatttta gtttaataag 1980

atgaagtaat aattctaaat ttccaggcat ttaaaaacat ggcctcaaaa tatatacttt 2040

taaaagttgt cagaactatg aaacaaatag aaaaattcac aaccctaatg gggaatttta 2100

tcacacttaa tgccagtcat taaagcagac aaaaatcagg aatgatctgg ataatttgag 2160

caacatgaat aacacaggaa gagcacttta ccttaccacc cctgaacaga catttttttc 2220

aagcacacat gaagcagtta ccaaagttga ccacatgcta ggccataaaa caagcttcaa 2280

cacttccacg agattgaatt catatagtct agtttttctg atcatgttat aattatgctg 2340

gaactcaaga gtaagaggat aactagaaaa ttcaaatagg tttggaagtt aggaaataca 2400

cttccgaaca acttattgcc aaagaagaaa gcatatgaa 2439

<210> 4
<211> 349

<212>  PRT
<213>  Homo sapiens

<400>  4

Met Pro Ala Asn Phe Thr Glu Gly Ser Phe Asp Ser Ser Gly Thr Gly
1               5                   10                  15

Gln Thr Leu Asp Ser Ser Pro Val Ala Cys Thr Glu Thr Val Thr Phe
            20                  25                  30

Thr Glu Val Val Glu Gly Lys Glu Trp Gly Ser Phe Tyr Tyr Ser Phe
        35                  40                  45

Lys Thr Glu Gln Leu Ile Thr Leu Trp Val Leu Phe Val Phe Thr Ile
    50                  55                  60

Val Gly Asn Ser Val Val Leu Phe Ser Thr Trp Arg Arg Lys Lys Lys
65                  70                  75                  80

Ser Arg Met Thr Phe Phe Val Thr Gln Leu Ala Ile Thr Asp Ser Phe
                85                  90                  95

Thr Gly Leu Val Asn Ile Leu Thr Asp Ile Ile Trp Arg Phe Thr Gly
            100                 105                 110

Asp Phe Thr Ala Pro Asp Leu Val Cys Arg Val Val Arg Tyr Leu Gln
        115                 120                 125

Val Val Leu Leu Tyr Ala Ser Thr Tyr Val Leu Val Ser Leu Ser Ile
    130                 135                 140

Asp Arg Tyr His Ala Ile Val Tyr Pro Met Lys Phe Leu Gln Gly Glu
145                 150                 155                 160

Lys Gln Ala Arg Val Leu Ile Val Ile Ala Trp Ser Leu Ser Phe Leu
                165                 170                 175

Phe Ser Ile Pro Thr Leu Ile Ile Phe Gly Lys Arg Thr Leu Ser Asn
            180                 185                 190

Gly Glu Val Gln Cys Trp Ala Leu Trp Pro Gly Asp Ser Tyr Trp Thr
        195                 200                 205

Pro Tyr Met Thr Ile Val Ala Phe Leu Val Tyr Phe Ile Pro Leu Thr
    210                 215                 220

Ile Ile Ser Ile Met Tyr Gly Ile Val Ile Arg Thr Ile Trp Ile Lys
225                 230                 235                 240

Ser Lys Thr Tyr Glu Thr Val Ile Ser Asn Cys Ser Asp Gly Lys Leu
                245                 250                 255

Cys Ser Ser Tyr Asn Arg Gly Leu Ile Ser Lys Ala Lys Ile Lys Ala
            260                 265                 270

Ile Lys Tyr Ser Ile Ile Ile Ile Leu Ala Phe Ile Cys Cys Trp Ser
        275                 280                 285

Pro Tyr Phe Leu Phe Asp Ile Leu Asp Asn Phe Asn Leu Leu Pro Asp
    290                 295                 300

Thr Gln Glu Arg Phe Tyr Ala Ser Val Ile Ile Gln Asn Leu Pro Ala
305                 310                 315                 320

Leu Trp Glu Ala Glu Glu Gly Gly Ser Pro Glu Phe Glu Gln Glu Phe
                325                 330                 335

Glu Thr Ile Pro Val Asn Met Val Lys Thr Arg Leu Tyr
            340                 345

<210>  5
<211>  21
<212>  DNA
<213>  Artificial Sequence

<220>
<223>  Description of the artificial sequence: Primer

<400>  5
aaattcacaa ccctaatggg g                                                21

<210>  6
<211>  22
<212>  DNA
<213>  Artificial Sequence

<220>
<223>  Description of the artificial sequence: Primer

<400>  6

tgtggtcaac tttggtaact gc                                              22

<210>  7
<211>  20
<212>  DNA
<213>  Artificial Sequence

<220>
<223>  Description of the artificial sequence: Primer

<400>  7
tcaacctcct tccagacacc                                                 20

## Description

The present invention relates to nucleic acids and polypeptides associated with colorectal lesions. The invention is more specifically related to a nucleic acid encoding a G-protein coupled receptor (GPCR) and the polypeptide transcribed thereof, the expression of which is significantly altered in association with colorectal cancer and other cancers. Another aspect of the nucleic acids disclosed herein are transcripts of the gene disclosed herein, that are differentially produced in tumors of the gastrointestinal tract. Furthermore the present invention provides a method for early diagnosis, prognosis and monitoring of the disease course and for therapy and vaccination of colorectal lesions.

In colorectal lesions as in most other tumors there is a strong correlation between the patients outcome following initial therapy and the stage at which the disease has been diagnosed. So the earlier the cancer could be detected the better are the chances for the patient to survive. Thus sensitive testing methods are required for detecting the tumors in early stages.

The most promising methods for early diagnosis of tumors are those involving molecular markers characteristic for tumor cells.

Colorectal cancer is a quite heterogeneous disease. Multiple regulators of the cell growth can be involved in the genesis of cancer. These regulatory elements of the cell cycle can be either positive regulators, named oncogenes when mutated, so that a transformed state is reached, or negative regulators, named tumor suppressor genes. The number of factors known to be involved in the regulation of the cell cycle and potentially being candidates for the development of cancer exceeds 100 up to know and is still increasing.

The molecules being involved in the emergence of the cancerous state of a cell can be used to discriminate between cancer cells and normal tissue. Thus cancerous tissue can be detected by detecting molecules characteristic for the cancer cells. This turns out to be sophisticated due to the large number of molecules potentially being involved in causing cancer.

For improved diagnosis of tumors there is a need for new marker molecules for use in diagnosis of cancers and especially of colorectal cancer, that enable for specific early detection and give the opportunity to treat the disorders at an early stage.

The present invention provides nucleic acids and polypeptides associated with colorectal cancer. According to the present invention theses molecules may be used as molecular markers that allow for comprehensive detection of cell proliferative disorders such as e.g. colorectal lesions even at early stages. Further more the inventive polypeptides and polynucleotides may be used for the detection and therapy of disorders characterized by abnormal proliferation of cells.

The present invention thus provides polypeptides and nucleic acids, the expression of which is significantly altered associated with colorectal lesions, that allow for enhanced prognosis and diagnosis of diseases associated with abnormal cell proliferation such as tumors. Furthermore the nucleic acids disclosed herein comprise transcripts arising from alternative splicing of genes, that do not occur in normal tissue in the extent they may be found in tumorous tissue.

In another aspect of the invention the nucleic acids and/or polypeptides disclosed herein alone or in combination with other molecules may be used for therapy and/or vaccination of disorders associated with abnormal cell proliferation such as tumors.

Yet another aspect of the present invention are pharmaceutical compositions containing polypeptides and/or polynucleotides disclosed herein alone or combination with one or more other therapeutic or diagnostic agents and/or carrier or adjuvant substances.

The present invention also provides kits such as diagnostic kits or research kits for the detection of the polynucleotides or polypeptides disclosed herein or comprising the polynucleotides or polypeptides disclosed herein or combinations thereof.

During the experiments leading to the present invention a gene encoding a G-protein coupled receptor (GPCR) was identified, the expression of which is associated with colorectal lesions. The present invention furthermore is based on the inventors findings shown in the examples 1- 3, that the level of expression of nucleic acids as well as of polypeptides transcribed from the marker gene presented herein in Figure 4 in samples allows to diagnose and grade disorders such as e.g. colorectal lesions associated with abnormal cell proliferation, to predict the course of the disease and to follow up the disease after initial therapy.

The present invention provides nucleic acids encoding a G-protein coupled receptor (GPCR) and the G-protein coupled receptor polypeptide, which is associated with colorectal cancer.

In one aspect the method according to the present invention is especially useful for early detection of disorders associated with abnormal cell proliferation such as e.g. colorectal lesions and for detection of disseminated tumor cells in the course of diagnosis of minimal residual disease. In this aspect the detection of the level of the nucleic acids and polypeptides presented herein may be used as a marker indicative of the presence or absence of cell proliferative disorders.

In another aspect of the present invention the nucleic acids or polypeptides disclosed herein may be used in the course of diagnosis of disorders associated with abnormal proliferation of cells in samples such as tumor resections, biopsies or the like. In this aspect the invention provides a method, that allows to build a strategy for the therapy of diseases according to their molecular properties. According to the present invention the level of said polypeptides and/or nucleic acids can be used as a molecular marker for prognosis, monitoring and the design of a strategy of tumor therapeutics.

It is yet another aspect of the present invention to provide methods for identification of molecules binding to the nucleic acids and polypeptides of the present invention as well as of activators and inhibitors of the expression of the genes of the present invention. Also a method for the identification of drug candidates for the therapy of proliferative disorders is provided.

The present invention provides tumor associated nucleic acids encoding a G-protein coupled receptor and the G-protein coupled receptor polypeptides characterized by the sequences given in Figure 4. Furthermore the present invention provides variants of the nucleic acids and polypeptides, which arise from tumor associated alternative splicing events.

Marker molecules as used in the present invention my comprise nucleic acids and polynucleotides. On the level of nucleic acids the marker molecules may be DNA or RNA comprising genomic DNA, cDNA, and RNA such as mRNA or hnRNA. In one preferred embodiment of the invention nucleic acids arising from particular differential splicing events may be marker molecules.

Expression as used according to the present invention may comprise for example expression of proteins. The transcription to RNA and thus the level of mRNA may also be understood to be expression according to the present invention.

The expression of a compound is said to be significantly altered according to the present invention, if the level of expression differs by more than 30%. The alteration of the expression may comprise for example elevated expression or reduced expression of said compound. Another aspect of the altered expression may be an alteration in a way, that the compound is expressed under non wild-type circumstances. This may comprise, that the compound is for example expressed in situations, that naturally suppress the expression, or is not expressed in situations, that naturally induce the expression of the compound.

Alteration of the expression as used herein may also comprise an alteration in the transcription pattern of a gene. E.g. the alteration of the transcription pattern may comprise alternative splicing of the gene. The alterations in the transcription pattern may influence the polypeptides translated from the altered transcripts or may be restricted to untranslated regions. The alteration in the transcription pattern of a gene may comprise use of novel exons in the transcripts, deletions of exons in the transcripts or the variation in the ratios of different splicing variants in cells. Thus alterations in transcriptional patterns of genes as used herein may comprise the production of nucleic acids such as e.g. mRNA, cDNA etc. containing additional stretches of nucleic acid sequences compared to wild type nucleic acids occurring in control tissues. Alternatively the nucleic acids produced by alternative splicing patterns may produce nucleic acids missing stretches of nucleic acid sequences present in wild type polynucleotides. The presence of additional stretches may occur simultaneously with the absence of original sequence-stretches in single transcripts. Alterations in the expression of genes as used in the context of the present invention may also comprise an alteration in the level of expression of splicing variants of genes. This may include increased or decreased expression of particular splicing variants as well as expression of variants not present in wild type tissue or the absence of expression of splicing variants present in wild type tissue. In one embodiment the alteration of the expression of the splicing variants may comprise the alteration of the ratios of different splicing variants in said tissue.

Nucleic acids as used in the context of the present invention are preferably polynucleotides or fragments thereof. Preferred polynucleotides comprise at least 20 consecutive nucleotides, preferably at least 30 consecutive nucleotides and more preferably at least 45 consecutive nucleotides, that are identical, share sequence homology or encode for identical, or homologous polypeptides, compared to the polypeptides associated with the proliferative disorders disclosed herein. The nucleic acids according to the present invention may also be complementary to any of said polynucleotides. Polynucleotides may for example include single-stranded (sense or antisense) or double-stranded molecules, and may be DNA (genomic, cDNA or synthetic) or RNA. RNA molecules comprise as well hnRNA (containing introns) as mRNA (not containing introns). According to the present invention the polynucleotides may also be linked to any other molecules, such as support materials or detection marker molecules, and may, but need not, contain additional coding or non-coding sequences.

The polynucleotides according to the present invention may be native sequences or variants thereof. The variants may contain one or more substitutions, additions, deletions and/or insertions such that the immunogenicity of the encoded polypeptide is not diminished, relative to the respective native proteins. The variants show preferably 70%, more preferably at least 80% and most preferably at least 90% of sequence identity to the native nucleic acid molecules used in the methods according to the present invention. Methods for determination of sequence similarity are known to those of skill in the art.

One example for detecting the similarity of sequences can be carried out using the FastA and/or BlastN bioinformatics software accessible on the HUSAR server of the DKFZ Heidelberg.

Furthermore nucleic acids according to the present invention are all polynucleotides, which hybridise to probes specific for the sequences disclosed herein under stringent conditions. Stringent conditions applied for the hybridisation reaction are known to those of ordinary skill in the art and may be applied as described in Sambrook et al. Molecular cloning: A Laboratory Manual, 2nd Edition, 1989.

The present invention also provides polynucleotides, that due to the degeneracy of the genetic code encode the polypeptides natively encoded by the disclosed nucleic acids while not showing the percentage of sequence homology as described above within the nucleic acid sequence. Such nucleic acids might arise by changing the codons present in the disclosed sequences by degenerate codons and so preparing a synthetic nucleic acid.

The nucleotide sequences according to the present invention may be joined to a variety of other nucleic acid sequences using the known recombinant DNA techniques. The sequences may for example be cloned into any of a variety of cloning vectors, such as plasmids, phagemids, lambda phage derivatives and cosmids. Furthermore vectors such as expression vectors, replication vectors, probe generation vectors and sequencing vectors may be joined with the sequences disclosed herein. Sequences of special interest, that could be cloned to the nucleic acids according to the present invention are for example non coding sequences and regulatory sequences including promoters, enhancers and terminators.

In a preferred embodiment polynucleotides may be formulated such, that they are able to enter mammalian cells and to be expressed in said cells. Such formulations are especially useful for therapeutic purposes. The expression of nucleic acid sequences in target cells may be achieved by any method known to those skilled in the art. The nucleic acids may for example be joined to elements that are apt to enable their expression in a host cell. Such elements may comprise promoters or enhancers, such as CMV-, SV40-, RSV-, metallothionein I- or polyhedrin-promotors respectively CMV- or SV40-enhancers. Possible methods for the expression are for example incorporation of the polynucleotides into a viral vector including adenovirus, adeno-associated virus, retrovirus, vaccinia virus or pox virus. Viral vectors for the purpose of expression of nucleic acids in mammalian host cells may comprise pcDNA3, pMSX, pKCR, pEFBOS, cDM8, pCEV4 etc.. These techniques are known to those skilled in the art.

Other formulations for administration in therapeutic purposes include colloidal dispersion systems such as for example macromolecule complexes, microspheres, beads, micelles and liposomes.

Generally, by means of conventional molecular biological processes it is possible (see, e.g., Sambrook et al., supra) to introduce different mutations into the nucleic acid molecules of the invention. As a result the inventive colorectal tumor associated polypeptides or polypeptids related thereto with possibly modified biological properties are synthesized. One possibility is the production of deletion mutants in which nucleic acid molecules are produced by continuous deletions from the 5'- or 3'-terminal of the coding DNA sequence and that lead to the synthesis of polypeptids that are shortened accordingly. Another possibility is the introduction of single-point mutation at positions where a modification of the amino aid sequence influences, e.g., the G-protein coupled receptor properties. By this method muteins can be produced, for example, that possess a modified binding affinities or that are no longer transduce signals in a regulation mechanisms that normally exist in the cell, e.g. with regard to binding of molecules to the receptor. Such muteins might also be valuable as therapeutically useful antagonists of the inventive colorectal tumor associated marker.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other, adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptides encoded by the various variants of the nucleic acid molecules of the invention show certain common characteristics, such as G-protein coupled receptor activity, activity in the regulation of cell proliferation and differentiation, molecular weight, immunological reactivity or conformation or physical properties like the electrophoretical mobilty, chromatographic behavior, sedimentation coefficients, solubility, spectroscopic properties, stability, pH optimum, temperature optimum.

The invention furthermore relates to vectors containing the inventive colorectal tumor associated nucleic acid molecules encoding a G-protein coupled receptor. Preferably, they are plasmids, cosmids, viruses, bacteriophages and other vectors usually used in the field of genetic engineering. Vectors suitable for use in the present invention include, but are not limited to the T7-based dual expression vectors (expression in prokaryotes and in eucaryotes) for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokaryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors of the invention. A host cell is understood to be an organism that is capable to take up in vitro recombinant DNA and, if the case may be, to synthesize the polypeptids encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a polypeptide exhibiting a biological property such as e.g. the receptor properties of the inventive colorectal lesion associated GPCR and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an antibody directed against the inventive colorectal lesion associated GPCR, or, e.g., the polypeptide can be substantially purified by the one-step method described in Smith and Johnson, Gene 67; 31-40 (1988). These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. These polypeptides are preferably in a substantially purified form.

Polypeptides as used in the present invention comprise at least an immunogenic portion of the inventive colorectal lesion associated GPCR proteins disclosed herein. The polypeptides may be of any length. Immunogenic portion as used above is a portion of a protein, that is recognized by a B-cell and/or T-cell surface antigen receptor. The immunogenic portions comprise at least 10 amino acid residues, more preferably at least 20 amino acid residues of the GPCR protein associated with a colorectal lesion. In a preferred embodiment of the present invention, particular domains of the proteins, such as for example transmembrane domains or N-terminal leader sequences have been deleted. The immunogenic portions according to the present invention react with antisera or specific antibodies in the same or nearly same intensity as the native full length proteins.

The immunogenic portions are generally identified using the techniques well known in the art. Possible techniques are for example screening of the polypeptides for the ability to react with antigen-specific antibodies, antisera and/or T-cell lines or clones.

The polypeptides associated with colorectal lesions according to the present invention comprise also variants of the native proteins. These variants may differ from the native protein in one or more alterations such as substitutions, deletions, additions and/or insertions. The immunoreactivity of the variants according to the present invention is not substantially diminished compared to the native proteins. In a preferred embodiment of the invention the immunoreactivity is diminished less than 50% in a more preferred embodiment the immunoreactivity is diminished less than 20 % compared to the native polypeptides.

In a preferred embodiment variants may be deficient in one or more portions, such as for example N-terminal leader sequences, transmembrane domains or small N- and/or C-terminal sequences. The variants exhibit 70%, more preferably at least 90% and most preferably at least 95% identity to the polypeptides disclosed according to the present invention.

The variants of the present invention are preferably conservative substitutions, so that the amino acids changed are substituted for amino acids with similar properties. The properties concerned may include polarity, charge, solubility, hydrophobicity, hydrophilicity and/or amphipathic nature of the amino acid residues. The variants disclosed herein may also comprise additional terminal leader sequences, linkers or sequences, which enable synthesis, purification or stability of the polypeptides in an easier or more comfortable way.

The polypeptides according to the present invention comprise also polypeptides that are fusion or chimeric polypeptides comprising the amino acid sequence encoded by the nucleic acid sequence of the inventive colorectal lesion associated GPCR disclosed herein. The polypeptides may be fused to any suitable amino acid sequences. These sequences may for example comprise antigenic fragments, receptors, enzymes, toxins, chelating epitopes, etc. In a preferred embodiment of the present invention the amino acid sequences, that are fused to the disclosed polypeptides are tags useful in the purification or recovery of the polypeptides such as e.g. his-tags or myc-tags. The amino acid sequences fused together may be directly linkes or may be separated by any linker or spacer sequences suitable in the particular purpose.

The polypeptides and polynucleotides according to the present invention are isolated. This means that the molecules are removed from their original environment. Naturally occurring proteins are isolated if they are separated from some or all of the materials, which coexist in the natural environment. Polynucleotides are isolated for example if they are cloned into vectors.

Furthermore the present invention provides binding agents such as antibodies and antigen-binding fragments, that specifically bind to the proteins associated with a colorectal lesions disclosed herein.

The term binding agent comprises a variety of substances such as oligopeptides, antibodies, peptdiomimetic molecules comprising antigen binding oligopeptides, nucleic acids, carbohydrates, organic compounds, etc.. Antibody according to the present invention preferably relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

Binding agents according to the present invention may for example be employed for the inhibition of the activity of the inventive colorectal lesion associated marker polypeptides disclosed herein. In this respect the term "binding agents" relates to agents specifically binding to the polypeptides transcribed from the novel colorectal lesion associated nucleic acids and thus inhibiting the activity of said polypeptide. Such binding agents may for example comprise nucleic acids (DNA, RNA, PNA etc.), polypeptides (antibodies, receptors, antigenic fragments, oligopeptides), carbohydrates, lipids, organic or inorganic compounds (metal-ions, sulfur compounds, boranes, silicates, reducing agents, oxidizing agents). The binding agents may preferably interact with the polypeptide by binding to epitopes, that are essential for the biological activity. The interaction may be reversible or irreversible. The binding may be noncovalent or even covalent binding to the polypeptide.

Furthermore the binding agents may introduce alterations to the polypeptide, that alter or diminish the biological activity of the inventive polypeptide.

For certain purposes, e.g. diagnostic methods, the antibody or binding agent of the present invention may be detectably labeled, for example, with a radioisotope, a bioluminescent compound, a chemiluminescent compound, a fluorescent compound, a metal chelate, or an enzyme. Furthermore any method suitable for the detection of the intermolecular interaction may be employed.

The antibody or antigen-binding agent is said to react specifically, if it reacts at a detectable level with a protein disclosed herein, and does not significantly react with other proteins. The antibodies according to the present invention may be monoclonal or polyclonal antibodies. Other molecules o capable of binding specifically may be for example antigen-binding fragments of antibodies such as Fab fragments, RNA molecules or polypeptides. According to the present invention binding agents may be used isolated or in combination. By means of combination it is possible to achieve a higher degree of sensitivity.

The antibodies useful for the methods according to the present invention may comprise further binding sites for either therapeutic agents or other polypeptides or may be coupled to said therapeutic agents or polypeptides. Therapeutic agents may comprise drugs, toxins, radio-nuclides and derivatives thereof. The agents may be coupled to the binding agents either directly or indirectly for example by a linker or carrier group. The linker group may for example function in order to enable the coupling reaction between binding agent and therapeutic or other agent or the linker may act as > a spacer between the distinct parts of the fusion molecule. The linker may also be cleavable under certain circumstances, so as to release the bound agent under said conditions. The therapeutic agents may be covalently coupled to carrier groups directly or via a linker group. The agent may also be non-covalently coupled to the carrier. Carriers that can be used according to the present invention are for example albumins, polypeptides, polysaccharides or liposomes.

The antibody used according to the present invention may be coupled to one or more agents. The multiple agents coupled to one antibody may be all of the same species or may be several different agents bound to one antibody.

The invention also relates to a transgenic non-human animal such as transgenic mouse, rats, hamsters, dogs, monkeys, rabbits, pigs, C. elegans and fish such as torpedo fish comprising a nucleic acid molecule or vector of the invention, preferably wherein said nucleic acid molecule or vector may be stably integrated into the genome of said non-human animal, preferably such that the presence of said nucleic acid molecule or vector leads to the expression of the inventive colorectal lesion associated marker polypeptide (or related polypeptide) of the invention, or may otherwise be transiently expressed within the non-human animal. Said animal may have one or several copies of the same or different nucleic acid molecules encoding one or several forms of the inventive colorectal lesion associated marker polypeptide or mutant forms thereof. This animal has numerous utilities, including as a research model for the regulation of cell proliferation and differentiation and therefore, presents a novel and valuable animal in the development of therapies, treatment, etc. for diseases caused by deficiency or failure of the inventive colorectal lesion associated marker protein involved in the development of cell proliferative disorders, e.g., colorectal lesions. Accordingly, in this instance, the non-human mammal is preferably a laboratory animal such as a mouse or rat.

Preferably, the transgenic non-human animal of the invention further comprises at least one inactivated wild type allele of the corresponding gene encoding the inventive colorectal lesion associated GPCR polypeptide. This embodiment allows for example the study of the interaction of various mutant forms of the inventive colorectal lesion associated marker polypeptides on the onset of the clinical symtoms of disease associated with the regulation of cell proliferation and differentiation. All the applications that have been herein before discussed with regard to a transgenic animal also apply to animals carrying two, three or more transgenes. It might be also desirable to inactivate the inventive colorectal lesion associated GPCR expression or function at a certain stage of development and/or life of the transgenic animal. This can be achieved by using, for example, tissue specific, developmental and/or cell regulated and/or inducible promoters which drive the expression of, e.g., an antisense nucleic acid or ribozyme directed against the RNA transcript encoding the inventive colorectal lesion associated marker encoding mRNA; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of the mutant inventive colorectal lesion associated protein may be controlled by such regulatory elements.

Furthermore, the invention also relates to a transgenic mammalian cell which contains (preferably stably integrated into its genome or transiently introduced) a nucleic acid molecule according to the invention or part thereof, wherein the transcription and/or expression of the nucleic acid molecule or part thereof leads to reduction of the synthesis of an inventive colorectal lesion associated marker protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product. In another preferred embodiment the native nucleic acid sequence coding for the inventive colorectal lesion associated marker polypeptide may be altered or substituted by a variant of said nucleic acid sequence, e.g. by means of recombination, thus rendering the inventive colorectal lesion associated GPCR non functional. Thus an organism lacking the inventive colorectal lesion associated GPCR activity may be produced according to knock out experiments.

The provision of the nucleic acid molecule according to the invention opens up the possibility to produce transgenic non-human animals with a reduced level of the inventive colorectal lesion associated marker protein as described above and, thus, with a defect in the regulation of cell proliferation and differentiation. Techniques how to achieve this are well known to the person skilled in the art. These include, for example, the expression of antisense-RNA, ribozymes, of molecules which combine antisense and ribozyme functions and/or of molecules which provide for a co-suppression effect. When using the antisense approach for reduction of the amount of the inventive colorectal lesion associated GPCR proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a inventive colorectal lesion associated marker protein. In this case the homology is preferably higher than 80%, particularly higher than 90% and still more preferably higher than 95%. The reduction of the synthesis of a polypeptide according to the invention in the transgenic mammalian cells can result in an alteration in, e.g., degradation of endogenous proteins. In transgenic animals comprising such cells this can lead to various physiological, developmental and/or morphological changes.

Thus, the present invention also relates to transgenic non-human animals comprising the above-described transgenic cells. These may show, for example, a defect in g-protein couples receptor mediated signal transduction, a deficiency in regulation of cell proliferation and/or differentiation compared to wild type animals due to the stable or transient presence of a foreign DNA resulting in at least one of the following features:
(a) disruption of (an) endogenous gene(s) encoding the inventive colorectal lesion associated GPCR;
(b) expression of at least one antisense RNA and/or ribozyme against a transcript comprising a nucleic acid molecule of the invention;
(c) expression of a sense and/or non-translatable mRNA of the nucleic acid molecule of the invention;
(d) expression of an antibody of the invention;
(e) incorporation of a functional or non-functional copy of the regulatory sequence of the invention; or
(f) incorporation of a recombinant DNA molecule or vector of the invention.

Methods for the production of a transgenic non-human animal of the present invention, preferably transgenic mouse, are well known to the person skilled in the art. Such methods, e.g., comprise the introduction of a nucleic acid molecule or vector of the invention into a germ cell, an embryonic cell, stem cell or an egg or a cell derived therefrom. The non-human animal can be used in accordance with a screening method of the invention described herein and may be a non-transgenic healthy animal, or may have a disorder, preferably a disorder caused by at least one mutation in the inventive colorectal lesion associated marker protein. Such transgenic animals are well suited for, e.g., pharmacological studies of drugs in connection with mutant forms of the above described inventive colorectal lesion associated marker polypeptide. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe, amplification techniques based on nucleic acids (e.g. PCR) etc.; see supra.

Another aspect of the present invention is a pharmaceutical composition for use in the treatment of disorders associated with abnormal cell proliferation. The polypeptides, polynucleotides and binding agents (esp. antibodies) according to the present invention may be incorporated into pharmaceutical or immunogenic compositions.

The pharmaceutical compositions may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according the parameters, such as age, sex, body weight etc. of the patient, known to those of skill in the art.

The pharmaceutical compositions comprise said compounds and a physiologically acceptable carrier. The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109.

A pharmaceutical composition or vaccine may for example contain DNA, that codes for one or more 5 polypeptides according to the present invention. The DNA may be administered in a way that allows the polypeptides to be generated in situ. Suitable expression systems are known to those skilled in the art. In another embodiment of the invention the nucleic acids may be for example anti-sense constructs. Pharmaceutical compositions may also comprise nucleic acid molecules expressible in a mammalian or human host system comprising a viral or other expression system for example an adenoviral vector system.

The nucleic acid may also be administered as a naked nucleic acid. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

Alternatively the pharmaceutical compositions may comprise one or more polypeptides. The polypeptides incorporated into pharmaceutical compositions may be the inventive colorectal lesion associated GPCR polypeptide. Optionally a pharmaceutical composition may comprise one or more further polypeptides

Polypeptides of the present invention or fragments thereof, that comprise an immunogenic portion of a inventive colorectal lesion associated protein, may be used in immunogenic compositions, wherein the polypeptide e.g. stimulates the patient's own immune response to tumor cells. A patient may be afflicted with disease, or may be free of detectable disease. Accordingly, the compounds disclosed herein may be used to treat cancer or to inhibit the development of cancer. The compounds may be administered either prior to or following a conventional treatment of tumors such as surgical removal of primary tumors, treatment by administration of radiotherapy, conventional chemotherapeutic methods or any other mode of treatment of the respective cancer or its precursors.

Immunogenic compositions such as vaccines may comprise one or more polypeptides and a non-specific immune-response enhancer, wherein the non-specific immune response enhancer is capable of eliciting or enhancing an immune response to an exogenous antigen. Any suitable immune-response enhancer may be employed in the vaccines of this invention. For example, an adjuvant may be included. Most adjuvants contain a substance designed to protect the antigen from rapid catabolism, such as aluminium hydroxide or mineral oil, and a non-specific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

Pharmaceutical compositions and vaccines may also contain other epitopes of tumor antigens, either incorporated into a fusion protein as described above (i.e., a single polypeptide that contains multiple epitopes) or present within a separate polypeptide.

Disorders characterized by abnormal cell proliferation, as used in the context of the present invention, may comprise for example neoplasms such as benign and malignant tumors, carcinomas, sarcomas, leukemias, lymhomas or dysplasias. Tumors may comprise tumors of the head and the neck, tumors of the respiratory tract, tumors of the gastrointestinal tract, tumors of the urinary system, tumors of the reproductive system, tumors of the endocrine system, tumors of the central and peripheral nervous system, tumors of the skin and its appendages, tumors of the soft tissues and bones, tumors of the lymphopoietic and hematopoietic system, cancer of the breast, cancer of the anogenital tract or cancer of the colorectal tract etc.. Tumors of the gastrointestinal tract may comprise e.g. tumors of the esophagus, tumors of the stomach, tumors of the liver, tumors of the pancreas, tumors of the bile, tumors of the small intestine, tumors of the colon or tumors of the rectum.

In one preferred embodiment of the invention the disorders are colorectal lesions, lesions of the respiratory tract or anogenital lesions. These lesions according to the present invention comprise conditions characterized by abnormal growth properties of cells or tissues compared to the growth properties of normal control cells or tissues. The growth of the cells or tissues may be for example abnormally accelerated or may be regulated abnormally. Abnormal regulation as used above may comprise any form of presence or absence of non wild-type responses of the cells or tissues to naturally occuring growth regulating influences. The abnormalities in growth of the cells or tissues may be for example neoplastic or hyperplastic. In one preferred embodiment of the invention the colorectal lesions are cancers or precancerours conditions of the colon.

A sample according to the method of the present invention is any sample, that may contain cells, tissues or body liquids. Furthermore any sample potentially containing the marker molecules to be detected may be a sample according to the present invention. Such samples are e.g. blood, plasma, serum, secretions, swabs, washes, stool, sputum, bile, cell- and tissue-samples or biopsies.

Biopsies as used in the context of the present invention may comprise e.g. resection samples of tumors, tissue samples prepared by endoscopic means or needle biopsies. Furthermore any sample potentially containing the marker molecules to be detected may be a sample according to the present invention.

The method for detection of the level of the polynucleotides or polypetides according to the present invention is any method, which is suited to detect very small amounts of specific molecules in samples. The detection reaction according to the present invention may be for example a detection either on the level of nucleic acids or on the level of polypeptides. The detection may either be a detection of the level of polypeptides or nucleic acids in cells in total or in cell lysats or a detection of the level of polypeptides or nucleic acids in distinct subcellular regions. The methods for determining the subcellular distribution of compounds are known to those of skill in the art. In one embodiment of the invention the detection of the marker molecules may comprise the detection of particular splicing variants. In another embodiment of the present invention the detection method may comprise the detection of methylation of nucleic acid molecules in samples.

Applicable formats for the detection reaction according to the present invention may be blotting techniques, such as Western-Blot, Southern-blot, Northern-blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as ELISA, RIA, lateral flow assays etc..

Methods for detection of methylation of nucleic acids are known to those of skill in the art and may comprise for example methods employing chemical pre-treatment of nucleic acids with e.g. sodium bisulphite, permanganate or hydrazine, and subsequent detection of the modification by means of specific restriction endonucleases or by means of specific probes e.g. in the course of an amplification reaction. The detection of methylation may furthermore be performed using methylation specific restriction endonucleases.

In one preferred embodiment of the invention the detection of the level of marker molecules is carried out by detection of the level of nucleic acids coding for the marker molecules or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognizing and binding to said nucleic acids. This method can be performed as well in vitro as directly in-situ for example in the course of a detecting staining reaction. Another way of detecting the marker molecules in a sample on the level of nucleic acids performed in the method according to the present invention is an amplification reaction of nucleic acids, which may be carried out in a quantitative manner such as for example PCR, LCR or NASBA.

In another preferred embodiment of the invention the detection of the level of marker molecules is carried out by determining the level of expression of a protein. The determination of the marker molecules on the protein level may for example be carried out in a reaction comprising a binding agent specific for the detection of the marker molecules. These binding agents may comprise for example antibodies and antigen-binding fragments, bifunctional hybrid antibodies, peptidomimetics containing minimal antigen-binding epitopes etc. The binding agents may be used in many different detection techniques for example in western-blot, ELISA, lateral flow assay, latex-agglutination, immunochromatographic strips or immuno-precipitation. Generally binding agent based detection may be carried out as well in vitro as directly in situ for example in the course of an immuno-cytochemical staining reaction. Any other method suitable for determining the amount of particular polypeptides in solutions of biological samples, such as biochemical, chemical, physical or physicochemical methods, can be used according to the present invention.

In one preferred embodiment of the invention the level of markers is significantly elevated compared to a non tumorous test sample. In this case the marker is overexpressed in the sample. In another preferred embodiment of the present invention the level of the marker is lowered compared to a non tumorous test sample. In a third embodiment there is no detectable expression of the marker at all in the test sample unlike in a control sample. In yet another embodiment there is detectable level of non wild-type marker molecules. Non wild-Type marker molecules may comprise any marker molecules that deviate in sequence or structure from the structure or sequence, that is functional in wild type tissue not affected by a cell proliferative disease. Wild type sequences or structures are the sequences or structures predominantly present in normal cells or tissues. In one preferred embodiment of the invention the level of particular splicing variants of the marker gene is altered in the test samples compared to the wild type tissue. This may lead to altered levels of splicing variants, new splicing variants, neo-peptides or altered ratios of different splicing variants of genes.

The detection of the level of molecular markers according to the present invention may be the detection of the level of single marker molecules in separated reaction mixtures as well as the detection of a combination of markers simultaneously. The combination may comprise the molecular markers disclosed herein and additionally further marker molecules useful for the detection of tumors.

The detection may be carried out in solution or using reagents fixed to a solid phase. The detection of one or more molecular markers may be performed in a single reaction mixture or in two or separate reaction mixtures. The detection reactions for several marker molecules may for example be performed simultaneously in multi-well reaction vessels. The markers characteristic for the colorectal lesions disclosed herein may be detected using reagents that specifically recognise these molecules. Simultaneously one or more further markers may be detected using reagents, that specifically recognize them. The detection reaction for each single marker may comprise one or more reactions with detecting agents either recognizing the initial marker molecules or preferably recognizing molecules used to recognize other molecules. Such reaction may e.g. comprise the use of primary and secondary and further antibodies. The detection reaction further may comprise a reporter reaction indicating the level of the inventive polypeptides associated with colorectal lesions. The reporter reaction may be for example a reaction producing a coloured compound, a bioluminescence reaction, a fluorescence reaction, generally a radiation emitting reaction etc..

In one preferred embodiment the detection of tissues expressing marker gene products is carried out in form of molecular imaging procedures. The respective procedures are known to those of ordinary skill in the art. Imaging methods for use in the context of the present invention may for example comprise MRI, SPECT, PET and other methods suitable for in vivo imaging.

In one embodiment the method may be based on the conversion of inert or labelled compounds to molecules detectable in the course of molecular imaging methods by the marker molecules. In another embodiment the molecular imaging method may be based on the use of compounds carrying a suitable label for in vivo molecular imaging, such as radio isotopes, metal ions etc., specifically binding to marker molecules in vivo.

In a preferred embodiment of the invention these compounds are non-toxic compounds and may be eliminated from the circulation of organisms, such as humans, in a time span, that allows for performing the detection of label accumulated in tumor tissue overexpressing the respective marker gene. In another preferred embodiment of the invention compounds are used for molecular imaging, for which clearance from the circulation is not relevant for performing the molecular imaging reaction.

This may be for example due to low background produced by the circulating molecules etc. The compounds for use in molecular imaging methods are administered in pharmaceutical acceptable form in compositions that may additionally comprise any other suitable substances, such as e.g. other diagnostically useful substances, therapeutically useful substances, carrier substances or the like.

The marker molecules disclosed according to the present invention may be used for diagnosis, monitoring of the disease course and prognosis in colorectal lesions.

Diagnosis of disorders associated with the expression of the inventive GPCR gene as used herein may for example comprise the detection of cells or tissues affected by abnormal growth. In one preferred embodiment diagnosis means the primary detection of a disease in an organism or sample. According to the present invention the method for diagnosis of disorders such as tumors may be applied in routine screening tests for preventive aspects in order to detect said disease at an early stage of the onset of the disorder. In another preferred embodiment the diagnostic method may be used to determine the minimal residual disease of a tumor after primary therapy. In this respect the method of the invention may be applied to determine cells in body samples displaying abnormal expression of marker molecules according to the present invention, characteristic for colorectal lesions. Thus a spread of affected cells may be detected in body liquids.

In one embodiment of the invention the methods disclosed herein may be used for the detection and identification of metastases. The method may be applied either for detection of metastases in body tissues or organs by the detection methods described herein, or the metastases may be diagnoses with respect to prognosis and prediction of disease course.

Monitoring of the disease course may comprise determining the levels of marker molecules at different time points, comparing the levels at the different time points and assessing a diagnosis about the progression of the disease over the covered period of time. Thus monitoring may enable for assessment of prognosis and/or for design of an adequate therapy for a particular patient.

Prognosis of the disease course of a disorders associated with abnormal cell proliferation according to the present invention may comprise determining the level of expression of one or more marker molecules, comparing the levels with data from subsequent studies in a database and prognosticating the disease course from said comparison. In a preferred embodiment the method may comprise the detection of the levels of a set of marker molecules, the distinct levels of which may characterize distinct stages in the course of the disease. In a further embodiment of the invention the combination of the levels of a combination of markers may be an indicator for the prognosis of the further disease course and may build the basis for design of an adequate therapy.

The present invention further provides kits for use in e.g. research or diagnostic methods. Such kits may contain components suitable for performing a scientific or diagnostic assay. Components may be compounds, reagents, containers and/or equipment. One component may be an antibody or fragment thereof that specifically binds to a polypeptide associated with colorectal lesions. Additionally the kit may contain reagents, buffers or others known in the art as necessary for performing the diagnostic assay. Alternatively the research kit or diagnostic kit may contain nucleotide probes or primers for the detection of DNA or RNA. Such a kit should contain appropriate additional reagents and buffers known in the art.

A kit according to present invention comprises:
a) reagents for the detection of the molecular marker molecules
b) the reagents and buffers commonly used for carrying out the detection reaction, such as buffers, detection-markers, carrier substances and others
d) a marker sample for carrying out a positive control reaction.
   The reagent for the detection of the marker includes any agent capable of binding to the marker molecule. Such reagents may include proteins, polypeptides, nucleic acids, glycoproteins, proteoglycans, polysaccharids or lipids.

The GPCR sample for carrying out a positive control may comprise for example nucleic acids in applicable form, such as solution or salt, peptides in applicable form, tissue section samples or positive cells expressing the GPCR.

In a preferred embodiment of the invention the detection of the marker molecules is carried out on the level of polypeptides. In this embodiment the binding agents may be for example antibodies specific for the marker molecules or fragments thereof.

In an other embodiment of the test kit the detection of the marker molecule is carried out on the nucleic acid level. In this embodiment of the invention the reagents for the detection may be for example nucleic acid probes or primers complementary to said marker molecule nucleic acids.

Another aspect of the present invention is to provide a method for therapy and/or vaccination. According to the present invention a therapy of cell proliferative disorders can be carried out using the inventive colorectal lesion associated polypeptides and/or polynucleotides. The therapy may be for example immunotherapy or somatic gene therapy.

The inventive colorectal lesion associated GPCR polypeptides and/or polynucleotides may according to the present invention be used for vaccination against cell proliferative disorders. Vaccination according to the present invention may comprise administering an immunogenic compound to an individual for the purpose of stimulating an immune response directed against said immunogenic compound and thus immunizing said individual against said immunogenic compound. Stimulating an immune response may comprise inducing the production of antibodies against said compound as well as stimulating cytotoxic T-cells. For the purpose of vaccination the polypeptides, nucleic acids and binding agents according to the present invention may be administered in a physiological acceptable form. The composition to be administered to individuals may comprise one or more antigenic components, physiologically acceptable carrier substances or buffer solutions, immunostimulants and/or adjuvants. Adjuvants may comprise for example Freund's incomplete adjuvant or Freund's complete adjuvant or other adjuvants known to those of skill in the art.

The composition may be administered in any applicable way such as e.g. intravenous, subcutaneous, intramuscular etc.. The dosage of the composition depends on the particular case and purpose of the vaccination. It has to be adapted to parameters by the individual treated such as age, weight, sex etc.. Furthermore the type of the immune response to be elicited has to be taken into account. In general it may be preferable if an individual receives 100 µg - 1 g of a polypeptide according to the present invention or 10⁶ - 10¹² MOI of a recombinant nucleic acid, containing a nucleic acid according to the present invention in a form that may be expressed in situ.

Individuals for the purpose of vaccination may be any organisms containing the inventive colorectal lesion associated polypeptides and/or polynucleotides and being able to get affected by cell proliferative disorders.

Vaccination of individuals may be favourable e.g. in the case of altered, non wild-type sequences or structure of marker molecules associated with cell proliferative disorders.

Polypeptides disclosed herein may also be employed in adoptive immunotherapy for the treatment of cancer. Adoptive immunotherapy may be broadly classified into either active or passive immunotherapy. In active immunotherapy, treatment relies on the in vivo stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the 5 cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T Cells: Principles Revisited," Immunological Reviews, 157:177, 1997).

The polypeptides disclosed herein may also be employed to generate and/or isolate tumor-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8⁺ CTL clones may be isolated from the patient, expanded using standard tissue culture techniques, and returned to the patient.

Alternatively, peptides corresponding to immunogenic portions of the polypeptides of the invention may be employed to generate tumor reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.™. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumor antigen-specific T-cells is then expanded using standard techniques and the cells are administered back to the patient.

In another embodiment, T-cell and/or antibody receptors specific for the polypeptides can be cloned, expanded, and transferred into other vectors or effector cells for use in adoptive immunotherapy. In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T-cells to provide antigen-specific T-cells, which may, in turn, be administered to a patient. The use of peptide-pulsed dendritic cells to generate antigen-specific T-cells and the subsequent use of such antigen-specific T-cells to eradicate tumors in a murine model has been demonstrated by Cheever et al, Immunological Reviews, 157:177, 1997.

Additionally, vectors expressing the disclosed nucleic acids may be introduced into stem cells taken from the patient and clonally propagated in vitro for autologous transplant back into the same patient.

Monoclonal antibodies of the present invention may also be used as therapeutic compounds in order to diminish or eliminate tumors. The antibodies may be used on their own (for instance, to inhibit metastases) or coupled to one or more therapeutic agents. Suitable agents in this regard include radio nuclides, differentiation inducers, drugs, toxins, and derivatives thereof. Preferred radio nuclides include 90Y, 1231, 1251, 1311, 186Re, 188Re, 211At, and 212Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

In one embodiment of the invention the therapy of disorders characterized by abnormal cell proliferaton may comprise the administration of antisense construct or ribozymes. The methods for administration of ribozymes or antisense constructs are known to those of skill in the art. The administration may take place as administration of naked nucleic acids or as administration of nucleic acids that are suited for expression of the relevant active products in situ.

In another embodiment of the invention the treatment of disorders may comprise the administration of binding agents directed against the GPCR polypeptides. These binding agents may for example be coupled to other compounds such as toxins, enzymes, radio-isotopes etc.

In another embodiment of the invention therapy of disorders associated with abnormal expression of the presented GPCR may comprise the administration of antagonists or agonists of the GPCR, of binding partners of the GPCR of inhibitors or enhancer of the expression of the inventive GPCR or of drugs identifiable by assays involving the measurement of the activity of the inventive GPCR. The methods for identifying these substances are known to those of skill in the art.

An example for a method for identifying a binding partner of an inventive colorectal lesion associated GPCR polypeptide (or related polypeptide) and/or polynucleotide may comprise:
(a) contacting the inventive colorectal lesion associated polypeptide of the invention with a compound to be screened; and
(b) determining whether the compound effects an activity of the polypeptide.

The inventive colorectal lesion associated GPCR may be used to screen for proteins or other o compounds that bind to the inventive colorectal lesion associated polypeptides or for proteins or other compounds to which the inventive colorectal lesion associated GPCR binds. The binding of the inventive colorectal lesion associated polypeptide and the molecule may activate (agonist), increase, inhibit (antagonist), or decrease activity of the inventive colorectal lesion associated polypeptide or the molecule bound. Examples of such molecules include antibodies, oligonucleotides, proteins (e.g., receptors), or small molecules.

Preferably, the molecule is closely related to the natural ligand of the inventive colorectal lesion associated GPCR, e.g., a fragment of the ligand, or a natural substrate, a ligand, a structural or functional mimetic; see, e.g., Coligan, Current Protocols in Immunology 1(2) (1991); Chapter 5. Similarly, the molecule can be closely related to a natural receptor to which the inventive colorectal lesion associated GPCR might bind, or at least, a fragment of the receptor capable of being bound by the inventive colorectal lesion associated polypeptide (e.g., active site). In either case, the molecule can be rationally designed using known techniques.

Preferably, the screening for these molecules involves producing appropriate cells which express the inventive colorectal lesion associated GPCR, either as a secreted protein or on the cell membrane. Preferred cells include cells from mammals, yeast, Drosophila, or E. coli. Cells expressing the inventive colorectal lesion associated GPCR (or cell membrane containing the expressed polypeptide) are then preferably contacted with a test compound potentially containing the molecule to observe binding, stimulation, or inhibition of activity of the inventive colorectal lesion associated GPCR.

The assay may simply test binding of a candidate compound to the inventive colorectal lesion associated GPCR, wherein binding is detected by a label, or in an assay involving competition with a labeled competitor. Further, the assay may test whether the candidate compound results in a signal generated by binding to the inventive colorectal lesion associated polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing the inventive colorectal lesion associated polypeptide, measuring the inventive colorectal lesion associated polypeptide/molecule activity or binding, and comparing the inventive colorectal lesion associated GPCR polypeptide/molecule activity or binding to a standard.

Preferably, an ELISA assay can measure the inventive colorectal lesion associated GPCR level or activity in a sample (e.g., biological sample) using a monoclonal or polyclonal antibody. The antibody can measure the inventive colorectal lesion associated polypeptide level or activity by either binding, directly or indirectly, to the inventive colorectal lesion associated polypeptide or by competing with the inventive colorectal lesion associated polypeptide for a substrate. All of these above assays can be used as diagnostic or prognostic markers. The molecules discovered using these assays can be used to treat disease or to bring about a particular result in a patient (e.g., elimination of a epthelial tumor or stop of progression of tumor growth) by activating or inhibiting the inventive colorectal lesion associated GPCR molecule. Moreover, the assays can discover agents which may inhibit or enhance the production of the inventive colorectal lesion associated polypeptide from suitably manipulated cells or tissues.

Therefore, the invention includes a method of identifying compounds for use in treatment of disorders characterized by abnormal cell proliferation which bind to a the inventive colorectal lesion associated GPCR comprising the steps of: (a) incubating a candidate binding compound with a polypeptide of the invention (the inventive colorectal lesion associated GPCR) ; and (b) determining if binding has occurred.

Moreover, the invention includes a method of identifying activators/agonists or inhibitors/antagonists of the inventive colorectal lesion associated GPCR for use in treatment of disorders characterized by abnormal cell proliferation comprising the steps of: (a) incubating a candidate compound with a polypeptide of the invention; b) assaying a biological activity, and (c) determining if a biological activity of the polypeptide of the invention (e.g. receptor activity) has been altered.

In a further embodiment, the present invention relates to method of identifying and obtaining a drug candidate for therapy of a disorder associated with abnormal cell proliferation comprising the steps of
(a) contacting a the inventive colorectal lesion associated GPCR or a cell expressing said polypeptide in the presence of components capable of providing a detectable signal in response
   to altered regulation of cell proliferation
   to altered activity of a GPCR
   to altered cell differentiation, with said drug candidate to be screened under conditions to allow protein degradation, and
(b) detecting presence or absence of a signal or increase of the signal generated from GPCR activity, cell proliferation or differentiation, wherein the presence or increase of the signal is indicative for a putative drug.

Experiments using animals or isolated cells or cell lines may be used to examine the proliferative behavior of cells or tissues in dependence on the inventive colorectal lesion associated polypeptide action. The same procedures may be employed for the study of cell differentiation.

The drug candidate may be a single compound or a plurality of compounds. The term "plurality of compounds" in a method of the invention is to be understood as a plurality of substances which may or may not be identical.

Said compound or plurality of compounds may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or activating the inventive colorectal lesion associated GPCR. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994) and in the appended examples. The plurality of compounds may be, e.g., added to the reaction mixture, culture medium, injected into a cell or otherwise applied to the transgenic animal. The cell or tissue that may be employed in the method of the invention preferably is a host cell, mammalian cell or non-human transgenic animal of the invention described in the embodiments hereinbefore.

If a sample containing a compound or a plurality of compounds is identified in the method of the invention, then it is either possible to isolate the compound from the original sample identified as containing the compound capable of suppressing or activating the inventive colorectal lesion associated GPCR, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical.

Several methods are known to the person skilled in the art for producing and screening large libraries to identify compounds having specific affinity for a target. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of the polypeptide of the invention and thus possible inhibitors and activators is described, for example, in Kramer, Methods Mol. Biol. 87 (1998), 25-39. This method can also be used, for example, for determining the binding sites and the recognition motifs in the polypeptide of the invention. In like manner, the substrate specificity of the DnaK chaperon was determined and the contact sites between human interleukin-6 and its receptor; see Rudiger, EMBO J. 16 (1997), 1501-1507 and Weiergraber, FEBS Lett. 379 (1996), 122-126, respectively. Furthermore, the above-mentioned methods can be used for the construction of binding supertopes derived from the polypeptide of the invention. A similar approach was successfully described for peptide antigens of the anti-p24 (HIV-1) monoclonal antibody; see Kramer, Cell 91 (1997), 799-809. A general route to fingerprint analyses of peptide-antibody interactions using the clustered amino acid peptide library was described in Kramer, Mol. Immunol. 32 (1995), 459-465. In addition, antagonists of the inventive colorectal lesion associated polypeptide of the invention can be derived and identified from monoclonal antibodies that specifically react with the polypeptide of the invention in accordance with the methods as described in Doring, Mol. Immunol. 31 (1994), 1059-1067.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the polypeptides according to the invention or nucleic acid molecules encoding such molecules are, for example, the in vitro screening with the phage display system as well as filter binding assays or "real time" measuring of interaction using, for example, the BIAcore apparatus (Pharmacia).

All these methods can be used in accordance with the present invention to identify activators/agonists and inhibitors/antagonists of the inventive colorectal lesion associated GPCR or related polypeptide of the invention.

Various sources for the basic structure of such an activator or inhibitor can be employed and comprise, for example, mimetic analogues of the polypeptide of the invention. Mimetic analogues of the polypeptide of the invention or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541.

Furthermore, in case fragments are used for the design of biologically active analogues pro-mimetic components can be incorporated into a peptide to re-establish at least some of the conformational properties that may have been lost upon removal of part of the original polypeptide; see, e.g., Nachman, Regul. Pept. 57 (1995), 359-370. Furthermore, the inventive colorectal lesion associated polypeptide can be used to identify synthetic chemical peptide mimetics that bind to or can function as a ligand, substrate, binding partner or the receptor of the polypeptide of the invention as effectively as does the natural polypeptide; see, e.g., Engleman, J. Clin. Invest. 99 (1997), 2284-2292. For example, folding simulations and computer redesign of structural motifs of the polypeptide of the invention can be performed using appropriate computer programs (Olszewski, Proteins 25 (1996), 286-299; Hoffman, Comput. Appl. Biosci. 11 (1995), 675-679). Computer modelling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the inventive colorectal lesion associated polypeptide and its possible ligand or other interacting proteins by computer assistant searches for complementary peptide sequences (Fassina, Immunomethods 5 (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptide mimetics of the protein of the invention or fragments thereof. Such pseudopeptide analogues of the natural amino acid sequence of the protein may very efficiently mimic the parent protein (Benkirane, J. Biol. Chem. 271 (1996), 33218-33224). For example, incorporation of easily available achiral *-amino acid residues into a protein of the invention or a fragment thereof results in the substitution of amide bonds by polymethylene units of an aliphatic chain, thereby providing a convenient strategy for constructing a peptide mimetic (Banerjee, Biopolymers 39 (1996), 769-777). Superactive peptidomimetic analogues of small peptide hormones in other systems are described in the prior art (Zhang, Biochem. Biophys. Res. Commun. 224 (1996), 327-331). Appropriate peptide mimetics of the protein of the present invention can also be identified by the synthesis of peptide mimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, a three-dimensional and/or crystallographic structure of the polypeptide of the invention can be used for the design of peptide mimetic inhibitors of the biological activity of the polypeptide of the invention (Rose, Biochemistry 35 (1996), 12933-12944; Rutenber, Bioorg. Med. Chem. 4 (1996), 1545-1558).

The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.

It is also well known to the person skilled in the art, that it is possible to design, synthesize and evaluate mimetics of small organic compounds that, for example, can act as a substrate or ligand to the inventive colorectal lesion associated polypeptide of the invention or the related polypeptide. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The nucleic acid molecule of the invention can also serve as a target for activators and inhibitors. Activators may comprise, for example, proteins that bind to the mRNA of a gene encoding a the inventive colorectal lesion associated polypeptide, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical and/or agricultural interest, and for identifying unknown RNA targets for use in treating a disease. These methods and compositions can be used in screening for novel antibiotics, bacteriostatics, or modifications thereof or for identifying compounds useful to alter expression levels of proteins encoded by a nucleic acid molecule. Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds which can act as antibiotics.

Some genetic changes lead to altered protein conformational states. For example, some mutant the inventive colorectal lesion associated polypetides may possess a tertiary structure that renders them far less capable of protein degradation. Restoring the normal or regulated conformation of mutated proteins is the most elegant and specific means to correct these molecular defects, although it may be difficult. Of particular interest in this regard is the consensus domain of the inventive colorectal lesion associated polypeptide described in the examples, below. Pharmacological manipulations thus may aim at restoration of wild-type conformation of the inventive colorectal lesion associated GPCR. Thus, the nucleic acid molecules and encoded polypeptides of the present invention may also be used to design and/or identify molecules which are capable of activating the wild-type, i.e. "GPCR" or "anti-GPCR" function of a the inventive colorectal lesion associated GPCR.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of the inventive colorectal lesion associated polypeptide and/or which exert their effects up- or downstream the inventive colorectal lesion associated GPCR may be identified using, for example, insertion mutagenesis using, for example, gene targeting vectors known in the art. Said compounds can also be functional derivatives or analogues of known inhibitors or activators. Such useful compounds can be for example transacting factors which bind to the inventive colorectal lesion associated GPCR or regulatory sequences of the gene encoding it. Identification of transacting factors can be carried out using standard methods in the art (see, e.g., Sambrook, supra, and Ausubel, supra). To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with the inventive colorectal lesion associated GPCR described above can also be achieved, for example, as described in Scofield (Science 274 (1996), 2063-2065) by use of the so-called yeast "two-hybrid system". In this system the polypeptide encoded by a nucleic acid molecule according to the invention or a smaller part thereof is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of a inventive colorectal lesion associated polypeptide of the invention, the complex is able to direct expression of the reporter gene. In this way the nucleic acid molecules according to the invention and the encoded peptide can be used to identify peptides and proteins interacting with the inventive colorectal lesion associated protein. It is apparent to the person skilled in the art that this and similar systems may then further be exploited for the identification of inhibitors of the binding of the inventive colorectal lesion associated proteins.

Once the transacting factor is identified, modulation of its binding to or regulation of expression of the inventive colorectal lesion associated GPCR can be pursued, beginning with, for example, screening for inhibitors against the binding of the transacting factor to the protein of the present invention. Activation or repression of the inventive colorectal lesion associated proteins could then be achieved in animals by applying the transacting factor (or its inhibitor) or the gene encoding it, e.g. in an expression vector. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene involved in the control of the inventive colorectal lesion associated polypeptide then can be identified. Modulation of the activities of these components can then be pursued, in order to develop additional drugs and methods for modulating the metabolism of protein degradation in animals. Thus, the present invention also relates to the use of the two-hybrid system as defined above for the identification of the inventive colorectal lesion associated polypeptide or activators or inhibitors of the inventive colorectal lesion associated polypeptide.

The compounds isolated by the above methods also serve as lead compounds for the development of analogue compounds. The analogues should have a stabilized electronic configuration and molecular conformation that allows key functional groups to be presented to the inventive colorectal lesion associated polypeptide or its possible receptor in substantially the same way as the lead compound. In particular, the analogue compounds have spatial electronic properties which are comparable to the binding region, but can be smaller molecules than the lead compound, frequently having a molecular weight below about 2 kD and preferably below about 1 kD. Identification of analogue compounds can be performed through use of techniques such as self-consistent field (SCF) analysis, configuration interaction (Cl) analysis, and normal mode dynamics analysis. Computer programs for implementing these techniques are available; e.g., Rein, Computer-Assisted Modelling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

In a preferred embodiment of the above-described methods of the invention said cell is a cell of or, obtained by a method of the invention or is comprised in the above-described transgenic non-human animal.

Once the described compound has been identified and obtained, it is preferably provided in a therapeutically acceptable form.

The present invention provides methods for detection and treatment of disorders characterized by abnormal cell proliferation, such as e.g. cancers. In one aspect the present invention provides a method for the detection of disorders characterized by abnormal cell proliferation, such as e.g. cancers based on the determination of the presence or absence and/or the level of expression of human the inventive colorectal lesion associated GPCR in biological samples. In a second aspect the present invention provides a method for treatment of disorders characterized by abnormal cell proliferation, such as e.g. cancers using the inventive colorectal lesion associated GPCR gene products as therapeutically active agents. The invention also provides for therapeutic methods based on the reduction of the enzymatic activity of the inventive colorectal lesion associated GPCR. It is one aspect of the invention to provide a method for rational tumor management based on the detection of the inventive colorectal lesion associated GPCR in patient samples and the tailoring of a therapy correlated to the detected overexpression of said gene products. Furthermore the present invention provides for a research or diagnostic test kit for performing the reactions involved in the detection of the presence or absence and/or the level of expression of the inventive colorectal lesion associated GPCR. Finally the present invention relates to pharmaceutical compositions applicable in the treatment of disorders according to the present invention.

### Brief description of the drawings

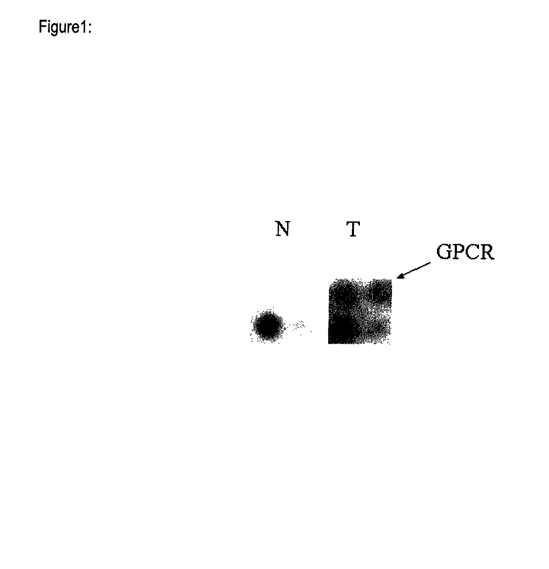

Figure 1: **Cloned Rsal restriction fragment of the inventive GPCR gene** that was spotted in duplicates on nylon membranes and hybridized with the normal colon subtracted probe (N) and the colon carcinoma subtracted probe (T), respectively.
Figure 2: **Results of RT PCR experiments** for determination of the level of expression of the inventive GPCR in four colon carcinoma samples (T) and the corresponding normal colon tissues (N). For experimental details see example 2.
Figure 3: **Gel electrophoretic analysis of the PCR products obtainable with primers specific for the inventive GPCR** In this experiment the various splicing variants (a-d) of the gene are displayed. For experimental details see example 3.
Figure 4: **Nucleic acid sequences and polypeptide sequences of the inventive GPCR**. A) Splicing variants (a), (b), and (c). B) Splicing variant (d) with altered polypeptide sequence. The primers used for the experiment in figure 3 are underlined.
Figure 5: **Overview of the alternative splicing events of the inventive GPCR**

The following examples are given for the purpose of illustration only and are not intended to limit the scope of the invention disclosed herein.

### Example 1: Identification of differential expression of genes in human colon tissue versus human colon tumor tissue

### (i) Isolation of total RNA:

Total RNA was extracted from human colon tissue by using the RNeasy kit (Qiagen) according to the manufacturer's protocol. The quality of the prepared RNA was determined by agarose gel electrophoresis according to the standard procedures (Sambrook J, et al., 1989). The mRNA fraction was amplified using the SMART™ PCR cDNA synthesis kit (Clontech).

### (ii) Identification of differentially expressed genes by suppressive subtractive hybridization:

This technique compares two populations of mRNA and provides clones of genes that are expressed in one population but not in the other. The applied technique was described in detail in Diatchenko et al., 1996. In the present invention, mRNA populations from human colon cancer tissue and normal colon tissue were compared. Specifically, mRNA of the normal colon biopsy was subtracted from mRNA of the colon cancer tissue. The necessary reagents were taken from the PCR-Select™ cDNA subtraction kit (Clontech) and all steps were performed as described in the manufacturer's protocol.

In detail, 2µg SMART™-amplified double-stranded cDNA was used. After Rsal-digestion and adaptor ligation hybridization of tester and driver were performed for 8 h (first hybridization) and 15 h (second hybridization) at 68°C. Two PCR steps were performed to amplify differentially expressed genes (first PCR: 27 cycles of 94°C 30 s, 66°C 30 s and 72°C 1.5 min; nested PCR: 12 cycles of 94°C 30 s, 66°C 30 s and 72°C 1.5 min) using adaptor specific primers (included in the subtraction kit) and 50x Advantage Polymerase Mix (Clontech). Efficiencies of Rsal-digestions, adaptor ligations and subtractive hybridizations were checked as recommended in the kit.

Subtracted cDNAs were inserted into the pCR2.1 vector and transformed into INVαF' cells (Invitrogen).

To isolate individual cDNAs of the subtracted library single bacterial transformants were incubated in 100 µl LB amp (50 µg/ml) at 37°C for at least 4 hours. Inserts were PCR amplified (95°C 30 sec, 68°C 3 min for 30 cycles) in 20 µl containing 10 mM Tris-HCI pH 9.0, 1.5 mM MgCl2, 50 mM KCI, 200 µM dNTP, 0.5 µM adaptor specific primers (included in the subtraction kit), 1.5 Units Taq polymerase (Pharmacia Biotech), and 1 µl of bacterial culture.

1.5 µl of a mixture containing 3 µl PCR amplified inserts and 2 µl 0.3 N NaOH/15% Ficoll were spotted onto a positively charged nylon membrane (Roche). In this way, hundreds of spots were arrayed on duplicate filters for subsequent hybridization. The differential screening step consists of hybridizations of the subtracted library with itself to minimize background (Wang et al., 1991). The probes were made of the nested PCR product of the subtraction following the instructions of the Clontech subtraction kit. Labeling with Digoxigenin was performed with the DIG DNA Labeling Kit (Roche). Hybridizations were carried out overnight in DIG Easy HYB (Roche) at 43°C. The filters were washed twice in 2 x SSC / 0.5% SDS at 68°C for 15 min and twice in 0,1 x SSC / 0.5% SDS at 68°C for 15 min, and subjected to detection using anti-DIG-AP conjugates and CDP-Star™ as chemiluminescent substrate according to the instructions of the DIG DNA Detection Kit (Roche). Blots were exposed to Kodak Biomax MR chemiluminescent film at room temperature for several minutes.

The GPCR encoding gene given in figure 4 has been identified to be differentially expressed in colorectal lesions compared to normal tissue. The GPCR gene is located on chromosome 7p14-15 (clone UWGC:g1564a307, AC005853).

### Example 2 : Detection of the level of expression of the inventive GPCR associated with colorectal disorders by RT-PCR in colorectal samples and samples of lung tissue

Samples of adenocarcinomas and adenomas of the colon and samples of lung carcinomas are used to determine the level of mRNA of the inventive GPCR using semi-quantitative RT PCR. Each 4 tumor biopsies (of the colon and of the lung) and samples of 4 adenomas are used in this study. Carcinomas and adenomas are collected, snap frozen, and stored at -80°C. They are verified to be composed predominantly of neoplastic cells by histopathological analysis. mRNA is isolated from tumors and patient-matched normal tissue using Qiagen reagents (Qiagen, Hilden, Germany), and single-stranded cDNA is synthesized using Superscript II (Life Technologies, Inc.). PCR is performed using the GeneAmp PCR System 9700 (PE Applied Biosystems) and the Titanium™ Taq PCR kit, as described in the manufacturers manual (Clontech Laboratories, Palo Alto, CA).

PCR reactions are performed in 25 µl volumes with a final concentration of 400nM for each primer (5'primer: 5'-AAATTCACAACCCTAATGGGG-3'; 3'primer: 5'-TGTGGTCAACTTTGGTAACTGC-3'), with 95°C for 30 sec, 60°C for 60 sec, and 68°C for 60 sec for 35 cycles.

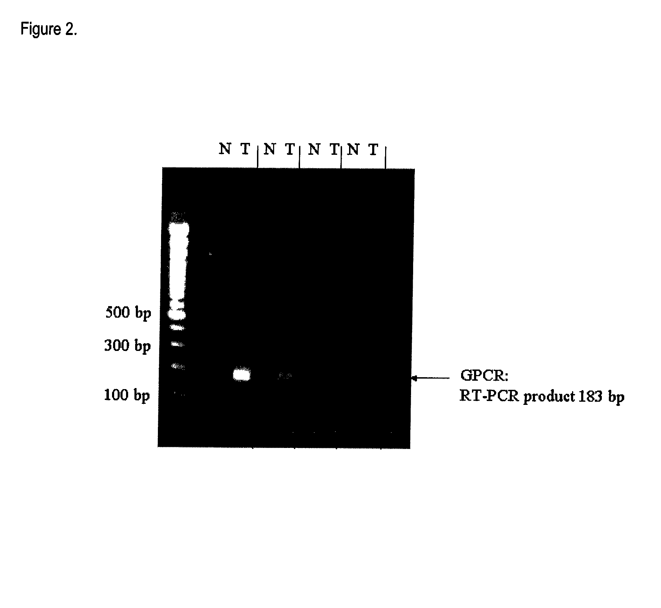

The specificity of the PCR products is verified by gel electrophoresis (figure 2).

The results show, that the gene identifiable by the method described in example 1 is overexpressed in tumor tissue in comparison to normal colon/lung mucosa tissue. Figure 2 shows the results of RT-PCR experiments for 4 different colon carcinomas.

The example illustrates, that the GPCR gene identified herein is suitable for the detection of disorders associated with abnormal proliferation of cells such as e.g. colorectal lesions or lung tumors by determining the expression level of the GPCR gene given in figure 4.

The present example illustrates that the comparison of the expression level of the particular marker molecules in test tissue to the expression level in normal control tissue may be used to state whether the test tissue is affected by a disorder or not. (Figure 2)

### Example 3: Detection of the splicing variants of the inventive GPCR

The GPCR transcript was found to be overexpressed in colon carcinoma biopsies when compared with normal colon by subtractive hybridization as described in example 1. The cloned Rsal restriction fragment of the GPCR is located in the 3' untranslated region of the GPCR gene. PCR is performed using the GeneAmp PCR System 9700 (PE Applied Biosystems) and the Advantage2 PCR kit, as described in the manufacturers manual (Clontech Laboratories, Palo Alto, CA).

PCR reactions are performed in 25 µl volumes with a final concentration of 400nM for each primer (5'primer: 5'-TCAACCTCCTTCCAGACACC-3'; 3'primer: 5'-TGTGGTCAACTTTGGTAACTGC'3'), with 95°C for 30 sec, 60°C for 60 sec, and 68°C for 4.5 min for 35 cycles.

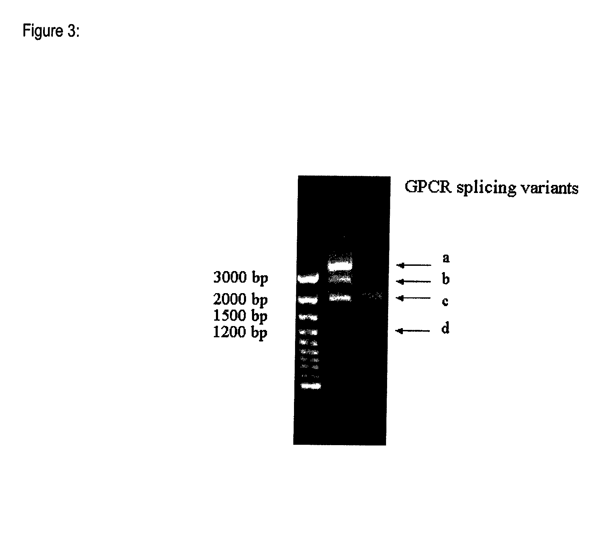

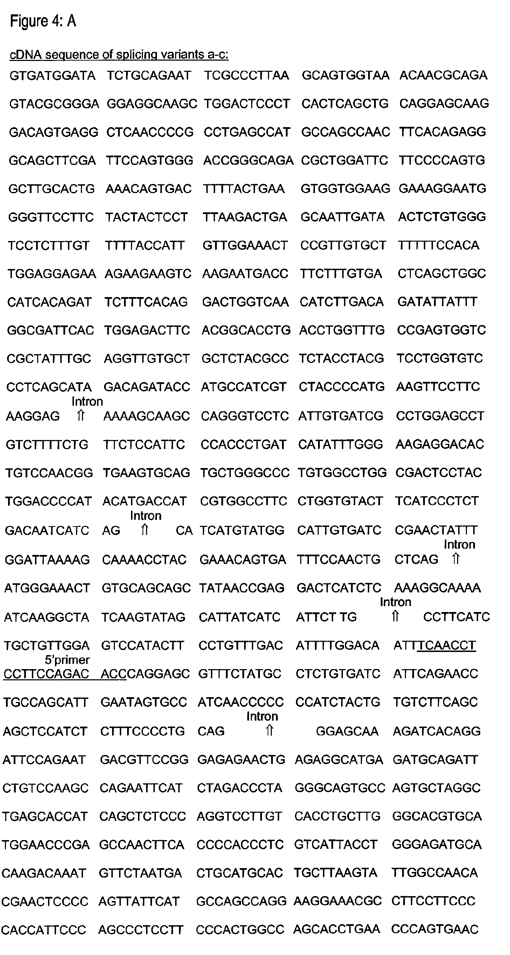

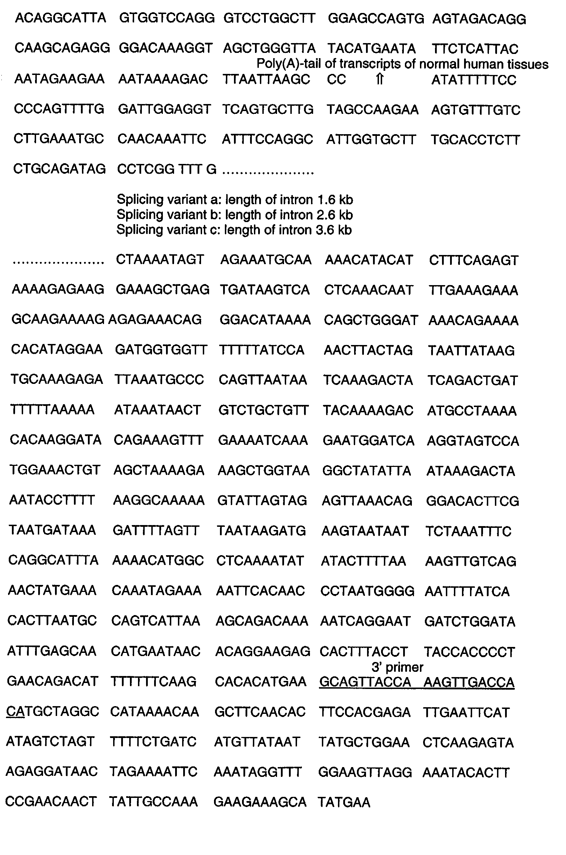

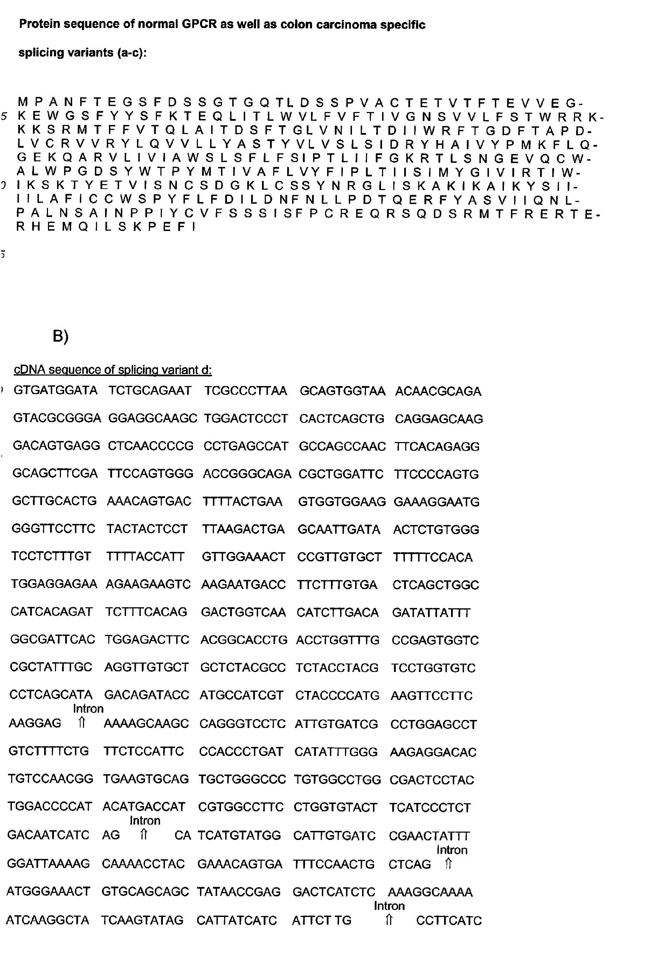

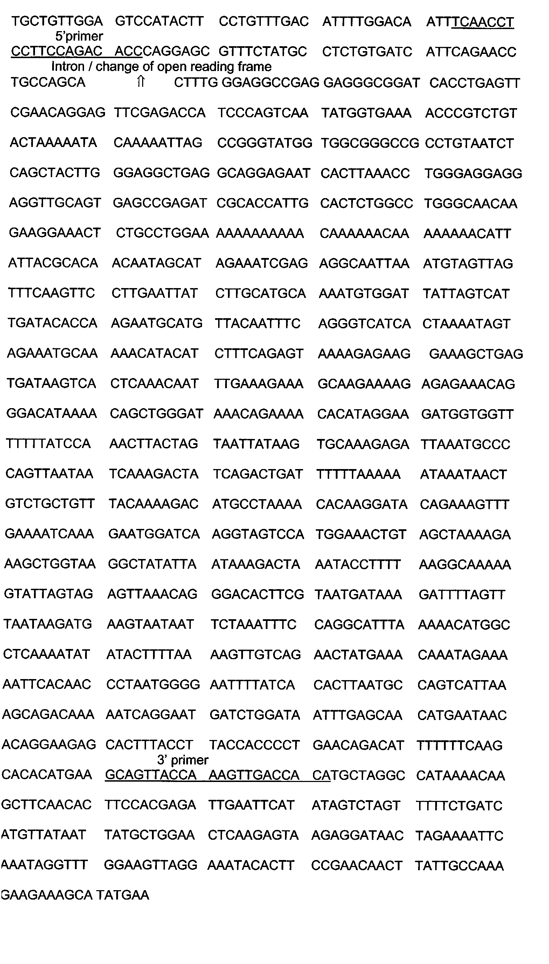

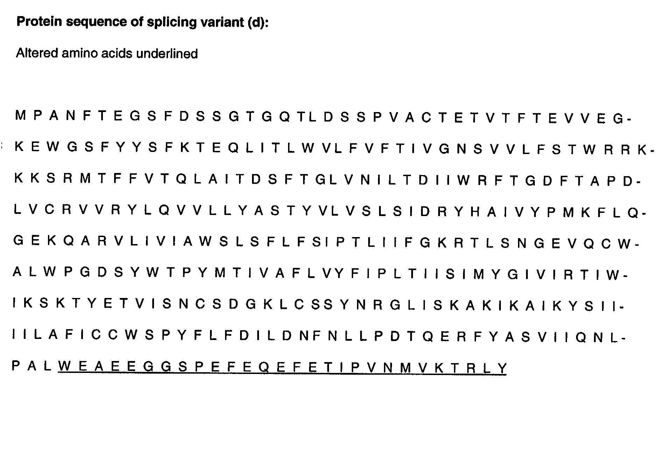

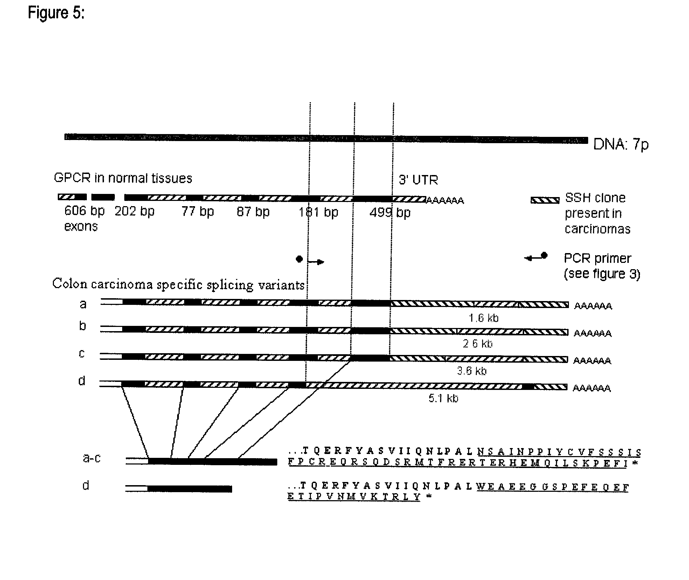

The PCR products are verified by gel electrophoresis (figure 3). The results show that four different PCR products are generated (a-d in figure 3). This indicates the presence of four different splicing variants int the test sample. For further analysis the PCR products are cloned into the pCR2.1 vector and transformed into INVαF' cells (Invitrogen). Individual bacterial transformants were incubated in 100 µl LB amp (50 µg/ml) at 37°C for at least 4 hours. Inserts were PCR amplified using 1µl of bacterial culture under conditions as described above. Positive clones containing the different splicing variants of the GPCR gene (a-d) were sequenced (MWG Biotech AG, Ebersberg, Germany) from both sides using primers as described above.

The results revealed that the colon carcinoma specific splicing variants (a-c) do not alter the amino acid sequence of the normal tissue protein (figure 4A). However, they represent transcripts with strongly elongated 3' untranslated regions which may lead to dysregulation of the GPCR gene in terms of protein expression, protein localization and protein function in carcinomas.

Splicing variant (d) alters the amino acid sequence of the normal tissue protein as the C-terminal 50 amino acids are replaced by 28 new amino acids due to a shift in the open reading frame (figure 4B). Carcinoma specific alteration of the intracellular domain of a GPCR may lead to dysfunction of the protein in terms of signal transduction, growth control, and apoptosis.

## Claims

1. An isolated nucleic acid molecule associated with colorectal lesions for use in the detection and therapy of disorders **characterized by** abnormal cell proliferation being selected from a group consisting of
a. a nucleic acid molecule encoding a polypeptide that comprises the amino acid sequence as depicted in Figure 4
b. a nucleic acid molecule comprising the nucleotide sequence as depicted in Figure 4;
c. a nucleic acid molecule encoding a polypeptide the sequence of which shows at least 65% identity to the amino acid sequence of the polypeptide encoded by a nucleic acid molecule specified in (a) or (b);
d. a nucleic acid molecule the sequence of which differs from the sequence of a nucleic acid molecule of (a) to (c) due to the degeneracy of the genetic code;
e. a nucleic acid molecule, which represents a fragment or an allelic variant of a nucleic acid molecule of (a) to (d);
f. a nucleic acid arising from an alternative splicing event of the gene presented in Figure 4; and
g. a nucleic acid, which encodes a fragment or a variant of the amino acid sequence depicted in Figure 4, which has an increased or decreased biological activity compared to the wild type protein showing the amino acid sequence.

2. An isolated polypeptide molecule associated with colorectal lesions for use in the detection and therapy of disorders **characterized by** abnormal proliferation of cells being selected from a group consisting of
a. a polypeptide, which is encoded by a nucleic acid molecule of claim 1.
b. a polypeptide, which comprises an amino acid sequence given in Figure 4;
c. a polypeptide, that is recognized by a binding agent, that has been raised against and is specifically binding the polypeptide of (a) or (b);
d. a fragment or a variant of the polypeptides of (a)-(c), that is encoded by a nucleic acid sequence, that hybridizes to a nucleic acid according to claim 1 under stringent conditions; and
e. a fragment or variant of the polypeptides of (a)-(d), which has an increased or decreased biological activity compared to the wild polypeptides associated with colorectal lesions.

3. A fusion polypeptide or chimeric nucleic acid for use in the detection or therapy of disorders **characterized by** abnormal proliferation of cells comprising a polypeptide or nucleic acid or fragments thereof according to claims 1 or 2.

4. A nucleic acid being DNA or RNA that is reverse complementary or complementary to a nucleic acid of claim 1 for use in diagnosis or therapy of disorders **characterized by** abnormal proliferation of cells.

5. A binding agent specifically recognizing and binding to a molecule of any one of the claims 1-4 for use in detection of disorders **characterized by** abnormal proliferation of cells.

6. A nucleic acid or a polypeptide according to any one of the claims 1-4 or the binding agent according to claim 5, which is detectably labelled.

7. A nucleic acid, a polypeptide or a binding agent according to claim 6, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

8. Method for diagnosis of disorders **characterized by** abnormal proliferation of cells and/or prognosis of disease course comprising
a. obtaining a sample from an individual
b. determining the levels of one or more marker molecules being nucleic acid molecules or polypeptides according to claim 1 or 2
c. comparing the levels of said nucleic acids or polypeptides within said sample to the contents within a corresponding test sample, not affected by the disease being tested,
wherein the diagnosis or prognosis of disease course is predicted from considering a significant change of the level of said marker molecule relative to the wild type level as indicative of said disorders **characterized by** abnormal proliferation of cells or of the disease course.

9. The method of claim 8, wherein the sample is a liquid containing nucleic acids, polypeptides or fragments thereof, a liquid containing cells or cell debris, a tissue sample, a cell sample or a biopsy.

10. The method of claim 8 or claim 9, wherein the sample is blood, plasma, serum, sputum, cell- and tissue-samples or biopsies.

11. The method according to any one of the claims 8-10, wherein the disorders **characterized by** abnormal proliferation of cells is a benign and malignant tumor, a carcinoma or a dysplasia.

12. The method according to claim 11, wherein the disorder **characterized by** abnormal proliferation of cells is cancer of the of the head and the neck, cancer of the respiratory tract, cancer of the gastrointestinal tract, cancer of the skin and its appendages, cancer of the central and peripheral nervous system, cancer of the urinary system,cancer of the reproductive system, cancer of the endocrine system, cancer of the soft tissues and bone, cancer of the lymphopoietic and hematopoietic system, cancer of the breast, cancer of the anogenital tract or cancer of the colorectal tract.

13. The method according to claims 8-12, wherein disorder **characterized by** abnormal proliferation of cells is a disorder of the respiratory tract, a disorder of the gastrointestinal tract or a disorder of the anogenital tract.

14. A method according to any one of the claims 8-13, wherein the detection of the expression of the marker molecules is carried out using at least one probe specifically binding to the marker molecules to be detected.

15. A method according to claim 14, wherein the probe is detectably labelled.

16. The method according to claim 15, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

17. The method according to any one of the claims 14-16, wherein at least one probe is a binding agent directed against said marker molecules.

18. The method of claim 17 wherein the binding agent is an antibody, a fragment of an antibody, a peptidomimetic comprising an antigen binding epitope or a mini-antibody.

19. The method according to any one of the claims 14-18, wherein the detection comprises an immuno-cytochemical detection procedure.

20. The method according to any one of the claims 14-16 , wherein at least one probe being a nucleic acid hybridising to a marker nucleic acid is used for the detection of the marker molecules.

21. The method according to claim 20, wherein the detection reaction comprises a nucleic acid amplification reaction.

22. The method according to claim 21, wherein the amplification reaction is PCR, LCR or NASBA.

23. The method according to any one of the claims 14-22, which is used for in-situ detection.

24. A method according to any one of the claims 8-23, which is used in the course of an in vivo or in vitro molecular imaging method.

25. A method according to any one of the claims 8-24, which is carried out in the course of early diagnosis of disorders, of minimal residual disease diagnosis or of preventive screening tests.

26. A test-kit for carrying out the method according to any one of the claims 8-25, comprising at least
a. reagents for the detection of polynucleotides and/or polypeptides
b. the reagents and buffers commonly used for carrying out the detection reaction, such as buffers, detection-markers, carrier substances and others

27. A test-kit according to claim 26, wherein the reagent for detection of the polynucleotide and/or polypeptide is an agent specifically binding to said polynucleotide and/or polypeptide.

28. Use of at least one component selected from a group consisting of
a. a nucleic acid according to claim 1 or claim 4;
b. a polypeptide according to claim 2 or claim 3;
c. a binding agent according to claim 5
d. an inhibitor or activator of the activity of the polypeptide according to claim 2
e. an activator or inhibitor of the expression of a nucleic acids according to claim 1
f. a binding partner of the polypeptide according to claim 2
g. and a drug or drug candidate identifiable using the nucleic acids and/or polypeptides according to claim 1 or 2 for the production of a medicament for treatment of disorders **characterized by** abnormal cell proliferation.

29. Use according to claim 28, wherein the disorder **characterized by** abnormal proliferation of cells is a benign and malignant tumor, a carcinoma or a dysplasia.

30. Use according to claim 29, wherein the disorder **characterized by** abnormal proliferation of cells is cancer of the head and the neck, cancer of the respiratory tract, cancer of the gastrointestinal tract, cancer of the skin and its appendages, cancer of the central and peripheral nervous system, cancer of the urinary system, cancer of the reproductive system, cancer of the endocrine system, cancer of the soft tissues and bone, cancer of the lymphopoietic and hematopoietic system, cancer of the breast, cancer of the anogenital tract or cancer of the colorectal tract.

31. Use according to claims 29 or 30, wherein disorder **characterized by** abnormal proliferation of cells is a disorder of the respiratory tract, a disorder of the gastrointestinal tract or a disorder of the anogenital tract.

32. A pharmaceutical composition for treatment of disorders **characterized by** abnormal proliferation of cells comprising at least one component selected from a group consisting of the following:
a. a nucleic acid according to claim 1 or claim 4;
b. a polypeptide according to claim 2 or claim 3;
c. a binding agent according to claim 5
d. an inhibitor or activator of the activity of the polypeptide according to claim 2
e. an activator or inhibitor of the expression of a nucleic acids according to claim 1
f. a binding partner of the polypeptide according to claim 2
g. and a drug or drug candidate identifiable using the nucleic acids and/or polypeptides according to claim 1 or 2.
